# EUROPEAN PATENT APPLICATION

(11) **EP 4 840 400 A1**
(43) Date of publication of application: **18.11.2026**
(21) Application number: 26174615.0
(22) Date of filing: 24.04.2026
(51) Int. Cl.: A61B 6/00, A61B 6/04

(54) **X-RAY DIAGNOSIS APPARATUS AND METHOD OF CONTROLLING X-RAY DIAGNOSIS APPARATUS**

(30) Priority: 28.04.2025 JP 2025074436; 03.04.2026 JP 2026061721
(71) Applicant: Canon Kabushiki Kaisha, Tokyo 146-8501 (JP)
(72) Inventor: NODA, Koji, Tokyo, 146-8501 (JP)
(74) Representative: Marks & Clerk LLP

(57) **Abstract**

An X-ray diagnosis apparatus according to an embodiment includes a C-arm, a C-arm support, a main arm, a top plate support arm, and processing circuitry. The C-arm supports an X-ray tube and an X-ray detector. The C-arm support rotatably supports the C-arm. The main arm is supported by a base, the main arm being configured to support the C-arm support. The top plate support arm is rotatable about an axis in a lateral direction of a top plate, the top plate support arm being supported by the main arm and being configured to support the top plate on which a subject is placed or the top plate support arm is supported by the main arm and to support a top plate on which a subject is placed, the top plate support arm including a plurality of support surfaces for supporting the top plate. The processing circuitry rotates the main arm by controlling the main arm.

## Description

### CROSS REFERENCE TO RELATED APPLICATIONS

This application is based upon and claims the benefit of priority from the prior Japanese Patent Application No. 2025-074436, filed on April 28, 2025, and No. 2026-061721, filed on April 3, 2026, the entire contents of which are incorporated herein by reference.

### FIELD

Embodiments described in the present specification and drawings relate to an X-ray diagnosis apparatus and a method of controlling X-ray diagnosis apparatus.

### BACKGROUND

Conventionally, among X-ray TV apparatuses including a C-arm used for multipurpose applications, there is a apparatus in which one end of a top plate on which a patient is placed is cantilevered by a rotatable top plate support arm. In such a apparatus, the top plate is supported by the top plate support arm in a type in which the top plate is supported from the right side and a type in which the top plate is supported from the left side when the X-ray TV apparatus is viewed from the front. These two types have a structure in which a user cannot freely change a support direction after installation of the X-ray TV apparatus because the user needs to select one of the types and manufacture the X-ray TV apparatus of the type selected by the user before the installation of the X-ray TV apparatus.

Further, in a case in which a patient seated in a wheelchair is imaged using the X-ray TV apparatus including the C-arm, sometimes the patient seated in the wheelchair is imaged while the top plate is detached, but the extra labor of the work of attaching and detaching the top plate is required before and after the imaging, so that there is a problem in that the convenience of the X-ray TV apparatus including the C-arm deteriorates while the efficient inspection workflow is hindered. Therefore, in the X-ray diagnosis apparatus, it is desired to change the support state of the top plate.

### SUMMARY OF INVENTION

An X-ray diagnosis apparatus comprising:
a C-arm configured to support an X-ray tube and an X-ray detector;
a C-arm support configured to rotatably support the C-arm;
a main arm configured to be supported by a base, the main arm being configured to support the C-arm support;
a top plate support arm configured to be rotatable about an axis in a lateral direction of a top plate, the top plate support arm being supported by the main arm and being configured to support the top plate on which a subject is placed; and
processing circuitry configured to rotate the main arm by controlling the main arm.

An X-ray diagnosis apparatus comprising:
a C-arm configured to support an X-ray tube and an X-ray detector;
a C-arm support configured to rotatably support the C-arm;
a main arm configured to be supported by a base, the main arm being configured to support the C-arm support;
a top plate support arm configured to be supported by the main arm and to support a top plate on which a subject is placed, the top plate support arm including a plurality of support surfaces for supporting the top plate; and
processing circuitry configured to rotate the main arm by controlling the main arm.

The top plate support arm may include:
a top plate support configured to be rotatable in the lateral direction of the top plate, the top plate support being configured to support the top plate; and/or
a connecting portion configured to connect the top plate support to the main arm.

The processing circuitry may be further configured to, in a first control mode for changing a support direction of the top plate, change the support direction of the top plate by rotating the main arm to move the top plate support arm in a left-to-right direction with respect to the base.

The processing circuitry may be further configured to, in the first control mode, move the top plate support arm in the left-to-right direction with respect to the base by rotating the main arm by 180°.

The processing circuitry may be further configured to, in the first control mode, move the top plate support arm in the left-to-right direction with respect to the base by rotating the main arm and rotating the top plate support arm about the axis in the lateral direction of the top plate.

The processing circuitry may be further configured to, in the first control mode, move the top plate support arm in the left-to-right direction with respect to the base by rotating the main arm and the top plate support arm in a first rotational direction while rotating the C-arm in a second rotational direction different from the first rotational direction.

The processing circuitry may be further configured to, in the first control mode, move the top plate support arm in the left-to-right direction with respect to the base by rotating the main arm in a first rotational direction while rotating the C-arm and the top plate support arm in a second rotational direction different from the first rotational direction.

The processing circuitry is further configured to execute the first control mode in a state in which the top plate is supported by the top plate support arm.

The processing circuitry may be further configured to:
determine whether the top plate support arm supports the top plate; and/or
rotate the main arm when the top plate support arm does not support the top plate.

The processing circuitry is further configured to, in the first control mode, move at least one of the main arm and the top plate support arm in a vertical direction to cause a height of the top plate after execution of the first control mode to substantially match a height of the top plate before the execution thereof.

The top plate support arm may include a fixer configured to detachably fix the top plate.

The processing circuitry may be further configured to, in a second control mode for retracting the top plate upward, move the top plate upward by executing rotation of the main arm in the lateral direction of the top plate and the rotation of the top plate support arm.

The processing circuitry may be further configured to, in the second control mode, move the top plate upward by executing rotation of the C-arm support in the lateral direction of the top plate and the rotation of the top plate support arm, and by executing movement of the C-arm along the main arm.

The processing circuitry may be further configured to, in the second control mode, move the top plate upward by rotating the C-arm support in a first rotational direction while rotating the C-arm and the top plate support arm in a second rotational direction different from the first rotational direction.

The processing circuitry may be further configured to execute the second control mode in a state in which the top plate is supported by the top plate support arm.

The processing circuitry may be further configured to, upon execution of the second control mode, move the top plate upward such that the top plate is held at a position higher than the base.

A method of controlling an X-ray diagnosis apparatus,
the X-ray diagnosis apparatus including:
a C-arm configured to support an X-ray tube and an X-ray detector;
a C-arm support configured to rotatably support the C-arm;
a main arm configured to be supported by a base, the main arm being configured to support the C-arm support; and
a top plate support arm configured to be rotatable about an axis in a lateral direction of a top plate, the top plate support arm being supported by the main arm and being configured to support the top plate on which a subject is placed,
the method comprising rotating the main arm by controlling the main arm.

The plurality of support surfaces may be provided symmetrically in a direction orthogonal to a longitudinal direction and the lateral direction of the top plate.

The top plate support arm may include a fixer configured to detachably fix the top plate,
the top plate support arm may include, as the plurality of support surfaces, a first support surface of the top plate support arm and a second support surface of the top plate support arm, and/or
the top plate support arm may have an insertion hole formed therein and configured for the fixer to be inserted thereinto, the fixer being configured to fix the top plate supported by the first support sur face when the top plate is supported by the first support surface, to fix the top plate supported by the second support surface when the top plate is supported by the second support surface, and to detachably fix the top plate.

The fixer may be a lock pin.

The X-ray diagnosis apparatus may comprise a locked state detection sensor configured to detect a locked state of fixation of the top plate by the lock pin; and/or
a top plate detection sensor configured to detect the top plate; and/or
the processing circuitry may be further configured to restrict rotation of the main arm when at least one of a case in which the locked state detection sensor detects a state in which the fixation of the top plate is unlocked and a case in which the top plate detection sensor detects a state in which the top plate is not attached is satisfied.

The processing circuitry may be further configured to determine control of at least one of the main arm and the top plate support arm according to dimensional information regarding dimensions of a space in which the X-ray diagnosis apparatus is disposed.

The processing circuitry may be further configured to:
determine whether the top plate support arm supports the top plate; and/or
issue a notification when the top plate support arm does not support the top plate.

A method of controlling an X-ray diagnosis apparatus,
the X-ray diagnosis apparatus including:
a C-arm configured to support an X-ray tube and an X-ray detector;
a C-arm support configured to rotatably support the C-arm;
a main arm configured to be supported by a base, the main arm being configured to support the C-arm support;
a top plate support arm configured to be supported by the main arm and to support a top plate on which a subject is placed, the top plate support arm including a plurality of support surfaces for supporting the top plate,
the method comprising rotating the main arm by controlling the main arm.

An X-ray diagnosis apparatus comprising:
a C-arm configured to support an X-ray tube and an X-ray detector;
a C-arm support configured to rotatably support the C-arm;
a main arm configured to be supported by a base, the main arm being configured to support the C-arm support;
a top plate support arm configured to be rotatable about an axis in a lateral direction of a top plate, the top plate support arm being supported by the main arm and being configured to support the top plate on which a subject is placed or a top plate support arm configured to be supported by the main arm and to support a top plate on which a subject is placed, the top plate support arm including a plurality of support surfaces for supporting the top plate; and
processing circuitry configured to rotate the main arm by controlling the main arm.

A method of controlling an X-ray diagnosis apparatus,
the X-ray diagnosis apparatus including:
a C-arm configured to support an X-ray tube and an X-ray detector;
a C-arm support configured to rotatably support the C-arm;
a main arm configured to be supported by a base, the main arm being configured to support the C-arm support;
a top plate support arm configured to be rotatable about an axis in a lateral direction of a top plate, the top plate support arm being supported by the main arm and being configured to support the top plate on which a subject is placed or a top plate support arm configured to be supported by the main arm and to support a top plate on which a subject is placed, the top plate support arm including a plurality of support surfaces for supporting the top plate,
the method comprising rotating the main arm by controlling the main arm.

### BRIEF DESCRIPTION OF THE DRAWINGS

FIG. 1 is a block diagram illustrating an example of a configuration of an X-ray diagnosis apparatus according to a first embodiment.
FIG. 2 is a perspective view illustrating an example of an external configuration of an imaging apparatus in the X-ray diagnosis apparatus according to the first embodiment.
FIG. 3A is a diagram illustrating an example of a detailed configuration of a top plate support arm according to the first embodiment.
FIG. 3B is a diagram illustrating an example of a detailed configuration of the top plate according to the first embodiment.
FIG. 4A is a diagram for ioescription of an example of arrangement of an operator and a peripheral apparatus in a case of performing gastrointestinal endoscopy.
FIG. 4B is a diagram for description of an example of arrangement of an operator and a peripheral apparatus in a case of performing Endoscopic Retrograde Cholangio Pancreatography (ERCP).
FIG. 5 is a flowchart for description of support direction change processing executed in the X-ray diagnosis apparatus according to the first embodiment.
FIG. 6 is a diagram illustrating an example of a support direction of the top plate before an input for a support direction change mode is received in the first embodiment.
FIG. 7 is a diagram illustrating an example of a support direction of the top plate after the input for the support direction change mode is received in the first embodiment.
FIG. 8 is a diagram illustrating an example of a case in which a top plate support in the top plate support arm is rotated in the first embodiment.
FIG. 9 is a diagram illustrating an example of the top plate support that supports the top plate in the first embodiment.
FIG. 10 is a diagram illustrating an example of the support direction of the top plate 24 after the support direction change mode is executed in the first embodiment.
FIG. 11 is a diagram illustrating the X-ray diagnosis apparatus before and after the support direction of the top plate is changed.
FIG. 12A is a diagram illustrating an example of a detailed configuration of a top plate support arm according to a first modification.
FIG. 12B is a diagram illustrating an example of a detailed configuration of a top plate according to the first modification.
FIG. 13 is a flowchart for description of support direction change processing executed in an X-ray diagnosis apparatus according to a second modification.
FIG. 14 is a diagram illustrating an example of a state in which a top plate, the attachment orientation of which has been changed, is attached to a top plate support arm in the second modification.
FIG. 15A is a diagram illustrating an example of a height of a top plate support arm from a floor surface before a support direction of a top plate is changed in a third modification.
FIG. 15B is a diagram illustrating an example of the height of the top plate support arm from the floor surface after the support direction is changed in the third modification.
FIG. 16 is a flowchart for description of support direction change processing executed in an X-ray diagnosis apparatus according to the third modification.
FIG. 17A is a diagram illustrating an example of a case in which a support direction of a top plate according to a second embodiment is on the right side of a base when the base is viewed from the front.
FIG. 17B is a diagram illustrating an example of a case in which the support direction of the top plate according to the second embodiment is on the left side of the base when the base is viewed from the front.
FIG. 18 is a flowchart for description of support direction change processing executed in an X-ray diagnosis apparatus according to the second embodiment.
FIG. 19A is a diagram illustrating an example of a detailed configuration of a top plate support arm according to a fourth modification.
FIG. 19B is a diagram illustrating an example of a detailed configuration of a top plate according to the fourth modification.
FIG. 20 is a diagram illustrating an example of a detailed configuration of a top plate support arm and a top plate according to a third embodiment.
FIG. 21 is a flowchart for description of support direction change processing executed in an X-ray diagnosis apparatus according to a fourth embodiment.
FIG. 22 is a diagram illustrating an example of a case in which a C-arm is moved to a predetermined position in the fourth embodiment.
FIG. 23 is a diagram illustrating an example of rotational operation of a main arm and a top plate support arm in the X-ray diagnosis apparatus according to the fourth embodiment.
FIG. 24 is a diagram illustrating an example of the orientation of a top plate after a support direction change mode is executed in the fourth embodiment.
FIG. 25 is a diagram illustrating an example in the middle of changing the orientation of the top plate in the fourth embodiment.
FIG. 26A is a diagram illustrating an example of a position of a top plate and a position of a C-arm when a base is viewed from the front after execution of a top plate retraction mode in a fifth embodiment.
FIG. 26B is a diagram illustrating an example of a position of the top plate and a position of the C-arm when the base is viewed from the side after execution of the top plate retraction mode in the fifth embodiment.
FIG. 27 is a flowchart for description of top plate retraction processing executed in an X-ray diagnosis apparatus according to the fifth embodiment.
FIG. 28A is a diagram illustrating an example of a positional relationship between the top plate and the C-arm after introduction of a subject in the fifth embodiment.
FIG. 28B is a diagram illustrating an example of a positional relationship between the top plate and the C-arm after introduction of the subject in the fifth embodiment.
FIG. 29 is a diagram illustrating an example of an aspect in which a top plate is disposed in a detachable state in a sixth embodiment.
FIG. 30 is a diagram illustrating an example of a state in which a cart for enabling the top plate to move is attached to the top plate in the sixth embodiment.
FIG. 31A is a diagram illustrating a state in which a first operation is performed by a user on a fixing mechanism in the sixth embodiment.
FIG. 31B is a diagram illustrating a state in which a second operation by the user is performed on the fixing mechanism in the sixth embodiment.
FIG. 31C is a diagram illustrating a state in which a third operation by the user is performed on the fixing mechanism in the sixth embodiment.
FIG. 32 is a diagram illustrating an example of a state in which the top plate is detached from the top plate support in the sixth embodiment.
FIG. 33 is a flowchart for description of support direction change processing executed in an X-ray diagnosis apparatus according to the sixth embodiment.
FIG. 34 is a flowchart for description of support direction change processing executed in an X-ray diagnosis apparatus according to a seventh embodiment.

### DETAILED DESCRIPTION

Hereinafter, respective embodiments of the X-ray diagnosis apparatus and the method of controlling X-ray diagnosis apparatus will be described with reference to the accompanying drawings. In the embodiments below, the same reference signs are given for identical components in terms of configuration and function, and duplicate description is omitted.

### [First Embodiment]

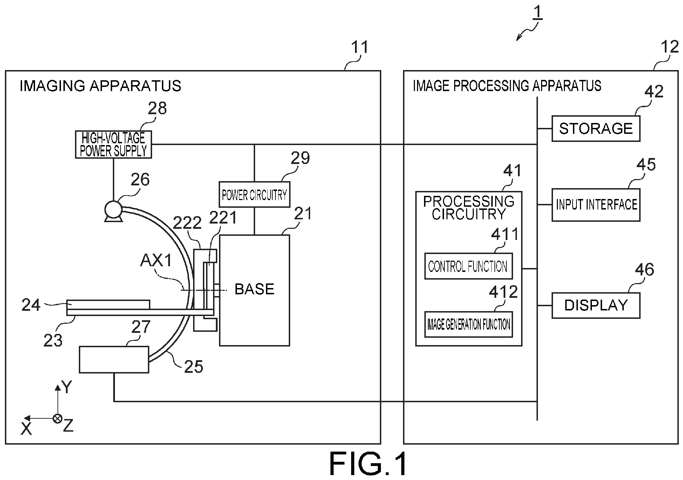

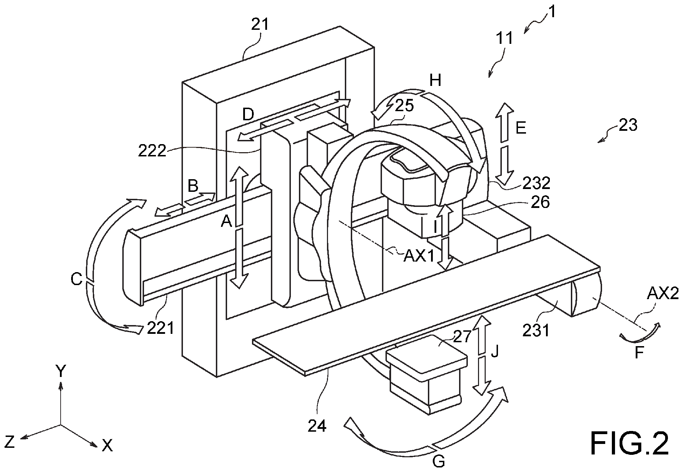

FIG. 1 is a block diagram illustrating a configuration example of an X-ray diagnosis apparatus according to a first embodiment. FIG. 2 is a perspective view illustrating an example of an external configuration of an imaging apparatus in the X-ray diagnosis apparatus according to the first embodiment.

FIGS. 1 and 2 illustrate an X-ray diagnosis apparatus 1 according to the first embodiment. The X-ray diagnosis apparatus 1 illustrated in FIGS. 1 and 2 is, for example, an X-ray TV apparatus including a C-arm. Note that the X-ray diagnosis apparatus 1 is not limited to the X-ray diagnosis apparatus 1 including the C-arm, and may be another type of X-ray diagnosis apparatus including the C-arm. As illustrated in FIG. 1, the X-ray diagnosis apparatus 1 includes an imaging apparatus 11 and an image processing apparatus 12. The imaging apparatus 11 is generally installed in a procedure room (also referred to as an examination room or a treatment room), while the image processing apparatus 12 is installed in a control room adjacent to the procedure room.

As illustrated in FIGS. 1 and 2, the imaging apparatus 11 includes a base 21, a main arm 221, a C-arm support 222, a top plate support arm 23, a top plate 24, a C-arm 25, an X-ray tube 26, an X-ray detector 27, a high-voltage power supply 28, and a power circuitry 29.

The base 21 is fixed to the floor. The main arm 221 is connected to the base 21, and the base 21 movably and rotatably supports the main arm 221.

The main arm 221 is supported by the base 21 and supports the C-arm support 222. Further, the main arm 221 is movably and rotatably supported with respect to the base 21. Specifically, the main arm 221 can slide so as to be vertically movable in an A direction with respect to the base 21 by the power circuitry 29. The main arm 221 can also move in a B direction with respect to the base by the power circuitry 29, and can move in the long axis direction of the top plate 24. The main arm 221 can be rotated in a C direction about a first axis AX1 parallel to an X-axis direction by the power circuitry 29.

The C-arm support 222 rotatably supports the C-arm 25. In addition, the C-arm support 222 is movably supported by the main arm 221. Specifically, the C-arm support 222 can slide in a D direction with respect to the main arm 221 by the power circuitry 29. As the C-arm support 222 slides with respect to the main arm 221, the C-arm 25 supported by the C-arm support 222 slides with respect to the main arm 221. The C-arm support 222 may be referred to as a C-arm holder.

The top plate support arm 23 is rotatable around an axis in the lateral direction of the top plate 24, is supported by the main arm 221, and supports the top plate 24. The top plate support arm 23 is supported by the main arm 221 in a cantilevered (or double-supported) manner. The top plate support arm 23 can be slid in an E direction with respect to the main arm 221 by the power circuitry 29. As illustrated in FIG. 2, the top plate support arm 23 includes a top plate support 231 and a connecting portion 232.

The top plate support 231 is rotatable in the lateral direction of the top plate 24 and supports the top plate 24. The top plate support 231 has a fixing mechanism that detachably fixes the top plate 24 to be described later. That is, the top plate support arm 23 has a fixing mechanism. Specifically, the top plate support 231 is rotatable in an F direction about a second axis AX2, which is an axis parallel to the lateral direction of the top plate 24, with respect to the connecting portion 232 by the power circuitry 29. When the top plate support 231 rotates in the F direction, the top plate 24 supported by the top plate support 231 also rotates integrally therewith.

The connecting portion 232 connects the top plate support 231 to the main arm 221. The connecting portion 232 is slidable with respect to the main arm 221 by the power circuitry 29. When the connecting portion 232 slides with respect to the main arm 221, the top plate support 231 also slides with respect to the main arm 221. In the example illustrated in FIG. 2, the connecting portion 232 supports the top plate support 231 at one end thereof.

A subject is placed on the top plate 24. The top plate 24 is supported by the top plate support 231 of the top plate support arm 23. Here, as illustrated in FIGS. 1 and 2, a vertical direction is defined as a Y-axis direction. When the top plate 24 is parallel to the floor surface (in the case illustrated in FIGS. 1 and 2), the lateral direction of the top plate 24 is defined as the X-axis direction, and the longitudinal direction thereof is defined as a Z-axis direction.

The C-arm 25 supports the X-ray tube 26 and the X-ray detector 27. Specifically, the C-arm 25 supports the X-ray tube 26 and the X-ray detector 27 so as to be arranged facing each other. The C-arm 25 can rotate in a G direction or rotate in an arc direction of the C-arm 25, that is, an H direction illustrated in FIG. 2, with respect to the C-arm support 222 by the power circuitry 29. The C-arm 25 can integrally rotate the X-ray tube 26 and the X-ray detector 27.

The X-ray tube 26 is provided at one end of the C-arm 25 and generates X-rays. The X-ray tube 26 can move forward and rearward in an I direction illustrated in FIG. 2 with respect to the X-ray detector 27 by the power circuitry 29. The X-ray tube 26 receives a supply of high-voltage power from the high-voltage power supply 28, and irradiates the X-ray detector 27 with X-rays according to a condition of the high-voltage power. The X-ray tube 26 may include, on the emission side, an X-ray irradiation field stop including a plurality of lead blades, a compensation filter formed of barium-containing silicon rubber or the like and configured to attenuate a predetermined amount of irradiation X-rays in order to prevent halation, and the like. As a result, the X-ray tube 26 can emit X-rays for imaging a predetermined region or X-rays for seeing through the predetermined region.

The X-ray detector 27 is provided at the other end of the C-arm 25 on the emission side of the X-ray tube 26, and detects X-rays emitted from the X-ray tube 26. The X-ray detector 27 can move forward and rearward in a J direction illustrated in FIG. 2 with respect to the X-ray tube 26 by the power circuitry 29. The X-ray detector 27 includes, for example, a flat panel detector (FPD) capable of detecting X-rays. The FPD includes a plurality of semiconductor detection elements. The semiconductor detection elements include an indirect conversion type and a direct conversion type. The indirect conversion type is a type in which incident X-rays are converted into light by a scintillator such as a phosphor, and the converted light is converted into an electrical signal. The direct conversion type is a type in which incident X-rays are directly converted into an electrical signal. An electrical signal generated by the plurality of semiconductor detection elements in association with the incidence of the X-rays is output to an analog to digital converter (A/D converter) (not illustrated). The A/D converter converts the electrical signal into digital data. The A/D converter outputs the digital data to a processing circuitry 41 of the image processing apparatus 12.

Note that the X-ray detector 27 may be an image intensifier (I.I.)-TV system. In the I.I.-TV system, X-rays transmitted through a patient and X-rays directly incident thereon are converted into visible light, luminance is further doubled in the process of optical-electronic-optical conversion to form projection data with high sensitivity, and optical projection data is converted into an electrical signal using a charge coupled device (CCD) imaging element.

The high-voltage power supply 28 supplies high-voltage power to the X-ray tube 26 under the control of the image processing apparatus 12. The high-voltage power supply 28 controls the X-ray tube according to X-ray conditions in pulse fluoroscopy. Here, the X-ray conditions include a tube voltage, a tube current, an irradiation time, and the like in the X-ray imaging.

The power circuitry 29 is a circuitry that drives the main arm 221, the C-arm support 222, the top plate support arm 23, the C-arm 25, the X-ray tube 26, and the X-ray detector 27 according to the control of the image processing apparatus 12.

The image processing apparatus 12 is configured based on a computer, and is a apparatus that performs operation control of the entire X-ray diagnosis apparatus 1, image processing on an X-ray image (X-ray image data) acquired by the imaging apparatus 11, and the like. The image processing apparatus 12 includes the processing circuitry 41, a storage 42, an input interface 45, and a display 46.

The processing circuitry 41 is an arithmetic circuitry that performs various operations, and includes, for example, a processor such as a central processing unit (CPU) or a graphics processing unit (GPU). The processing circuitry 41 controls, for example, the main arm 221 and generates medical image data. The processing circuitry 41 has a control function 411 and an image generation function 412. The control function 411 corresponds to a control unit according to the present embodiment, and the image generation function 412 corresponds to an image generation unit according to the present embodiment.

In the embodiment of FIG. 1, the control function 411 and each processing function performed by the image generation function 412 are stored in the storage 42 in the form of a program executable by a computer. The processing circuitry 41 is a processor that implements a function corresponding to each program by reading and executing the program from the storage 42. Note that, in FIG. 1, it has been described that the control function 411 and the image generation function 412 are implemented by the single processing circuitry 41, but these functions may be implemented by combining a plurality of independent processors to form the processing circuitry 41 and executing a program by each processor.

The control function 411 controls the main arm 221, the top plate support arm 23, the C-arm 25, the X-ray tube 26, and the X-ray detector 27 by controlling the high-voltage power supply 28 and the power circuitry 29. Specifically, the control function 411 controls the main arm 221 to rotate the main arm 221. More specifically, in the present embodiment, the control function 411 controls the main arm 221 and the top plate support arm 23 to rotate the main arm 221 and rotate the top plate support arm 23 about the axis in the lateral direction of the top plate 24. As a result, the control function 411 executes various imaging modes such as an X-ray imaging mode and an X-ray fluoroscopy mode.

The control function 411 executes a support direction change mode. The support direction change mode is a mode for changing the support direction of the top plate 24. Specifically, in the support direction change mode, the control function 411 changes the support direction of the top plate 24 by rotating the main arm 221 and moving the top plate support arm 23 in a left-to-right direction with respect to the base 21. The support direction change mode corresponds to a first control mode in the present embodiment.

The image generation function 412 generates image data based on the output from the X-ray detector 27. The image data is medical image data including a fluoroscopic image and a captured image related to the subject.

The storage 42 includes a random access memory (RAM), a semiconductor memory element such as a flash memory, a hard disk, an optical disk, and the like. The storage 42 may include a portable medium such as a universal serial bus (USB) memory and a digital video disk (DVD). The storage 42 stores various processing programs (in addition to the application program, an operating system (OS) and the like are also included) used in the processing circuitry 41, data necessary for executing the programs, and an X-ray image. In addition, the OS can include a graphical user interface (GUI) that uses graphics a lot to display information on the display 46 for the operator and can perform basic operations with the input interface 45.

The input interface 45 is a circuitry that inputs a signal from an input device operable by an operator, and here, the input device itself is also included in the input interface 45. The input interface 45 includes a switch, a pointing device (for example, a mouse), a keyboard, various buttons, and the like. When the operator operates the input interface 45, the input interface 45 generates an input signal corresponding to the operation and outputs the input signal to the processing circuitry 41. Note that the image processing apparatus 12 may include a touch panel in which the input interface 45 is integrated with the display 46.

The display 46 is a display device such as a liquid crystal display panel, a plasma display panel, or an organic electro luminescence (EL) panel.

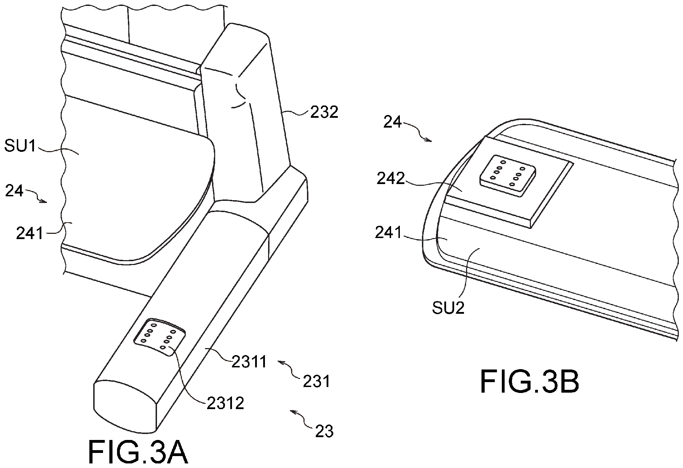

FIG. 3 is a diagram illustrating an example of a detailed configuration of the top plate support arm 23 and the top plate 24 according to the first embodiment. FIG. 3A is a diagram illustrating an example of a detailed configuration of the top plate support arm 23 according to the first embodiment, and FIG. 3B is a diagram illustrating an example of a detailed configuration of the top plate 24 according to the first embodiment. As illustrated in FIG. 3A, the top plate support 231 of the top plate support arm 23 includes a top plate support main body 2311 and a fixing mechanism 2312. As illustrated in FIG. 3B, the top plate 24 includes a top plate main body 241 and an attachment portion 242.

One end of the top plate support main body 2311 is connected to the connecting portion 232 so as to be rotatable in the F direction illustrated in FIG. 2 with respect to the connecting portion 232. As illustrated in FIG. 3, the top plate support main body 2311 is provided with the fixing mechanism 2312.

The fixing mechanism 2312 is a mechanism for fixing the top plate 24 to the top plate support main body 2311. Specifically, the fixing mechanism 2312 includes a locking mechanism (not illustrated), and fixes the attachment portion 242 of the top plate 24 by the locking mechanism, thereby fixing the top plate 24 to the top plate support 231 by fixing the top plate main body 241 of the top plate 24 to the top plate support main body 2311. Further, the fixing mechanism 2312 releases the lock of the attachment portion 242 of the top plate 24 by the locking mechanism, so that the top plate main body 241 of the top plate 24 can be detached from the top plate support main body 2311. That is, the fixing mechanism 2312 detachably fixes the top plate 24.

The top plate main body 241 is a plate-like member on which the subject is placed. The attachment portion 242 is a mechanism for attaching the top plate main body 241 to the fixing mechanism 2312. In the example illustrated in FIGS. 3A and 3B, the attachment portion 242 is provided on a second top plate surface SU2 of the top plate main body 241, which is located on the side opposite to a first top plate surface SU1.

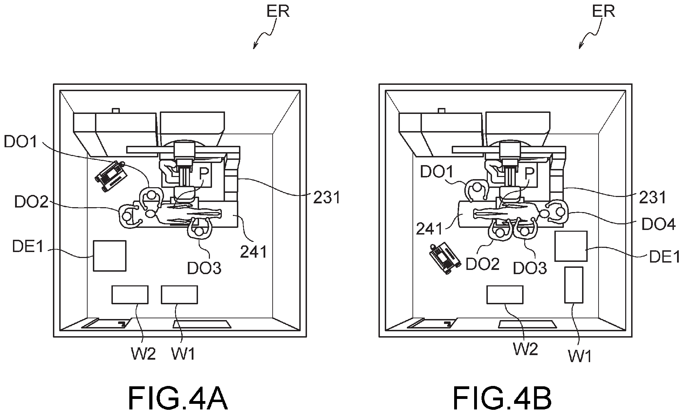

FIG. 4 is a diagram for description of an example of the arrangement of an operator and a peripheral device according to a procedure. FIG. 4A is a diagram for description of an example of the arrangement of an operator and a peripheral device in a case of performing gastrointestinal endoscopy in the examination room ER. FIG. 4B is a diagram for description of an example of the arrangement of an operator and a peripheral device in a case of performing Endoscopic Retrograde Cholangio Pancreatography (ERCP) in the examination room ER.

In each of the examples illustrated in FIGS. 4A and 4B, the top plate support arm 23 supports the top plate 24 from the right side with respect to the base 21. As illustrated in FIG. 4A, when gastrointestinal endoscopy is performed, operators DO1 to DO3 need to approach from the right-hand side of a subject P. Therefore, the operators DO1 to DO3 place the subject P on the top plate 24 such that the head of the subject P is placed on a side opposite to the side where the top plate support arm 23 supports the top plate 24. In this case, since a space is generated around the top plate 24 centered on the head of the subject P, an endoscope camera device DE1, an inspection device DE2, and wagons W1 and W2 on which instruments and the like necessary for the inspection are placed can be arranged, and the accessibility to the subject P by the operators DO1 to DO3 is also good, so that the workability of the clinical procedure can be improved.

On the other hand, as illustrated in FIG. 4B, when ERCP is performed, operators DO1 to DO4 need to approach from the left-hand side of the subject P. As described above, in ERCP, the operators DO1 to DO4 approach from the left side of the subject P, whereby the accessibility in a state in which the subject P is in the left decubitus position is improved when the endoscope camera is advanced from the oral cavity of the subject P to the duodenum. Therefore, the operators DO1 to DO4 place the subject P on the top plate 24 such that the head of the subject P is placed on a side where the top plate support arm 23 supports the top plate 24. However, since the top plate support arm 23 is arranged around the top plate 24 centered on the head of the subject P, the space is reduced, and the workability of the procedure deteriorates as compared with a case in which the head of the subject P is placed on the side opposite to the side where the top plate support arm 23 supports the top plate 24.

Therefore, in the example illustrated in FIG. 4B, in order to secure the space around the top plate 24 centered on the head of the subject P while the operators DO approach from the left-hand side of the subject P, it is necessary to change the support direction of the top plate 24 so that the top plate support arm 23 can support the top plate 24 from the left side with respect to the base 21.

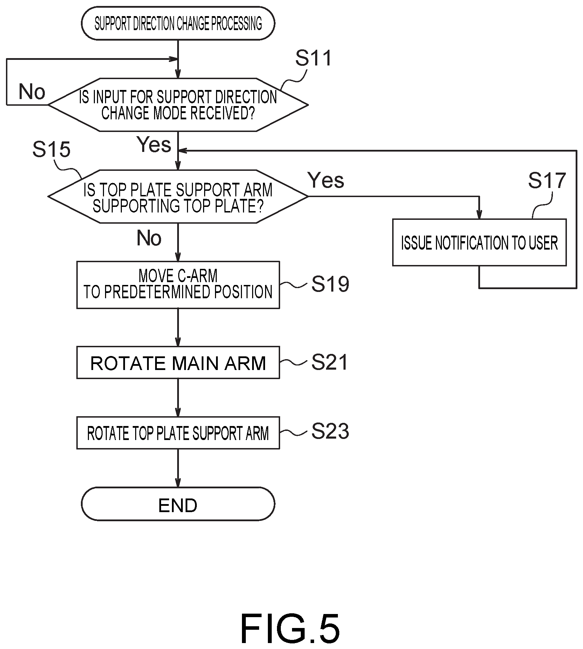

FIG. 5 is a flowchart for description of support direction change processing executed in the X-ray diagnosis apparatus 1 according to the first embodiment. In the support direction change processing, an input for the support direction change mode is received, the C-arm 25 is moved, the main arm 221 is rotated, and the top plate support arm 23 is rotated. For example, the support direction change processing is processing executed when the input for the support direction change mode is received.

As illustrated in FIG. 5, first, the control function 411 in the processing circuitry 41 of the X-ray diagnosis apparatus 1 determines whether an input for the support direction change mode has been received (step S11). Specifically, the control function 411 determines whether the input for the support direction change mode has been received from the operator DO via the input interface 45. Then, in step S11, when the control function 411 has not received the input for the support direction change mode (step S11: No), the control function 411 waits until the input for the support direction change mode is received.

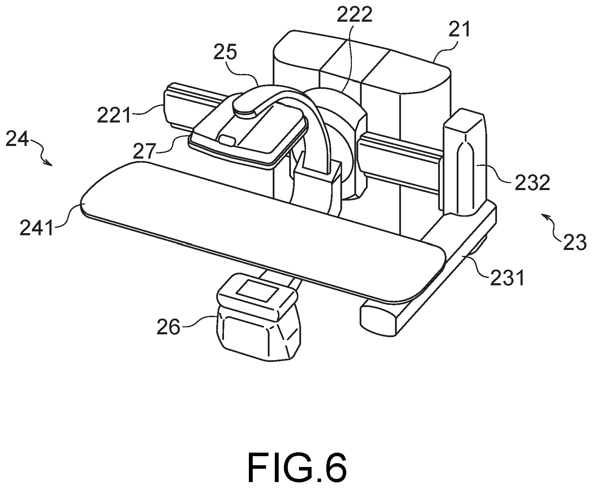

FIG. 6 is a diagram illustrating an example of the support direction of the top plate 24 before receiving the input for the support direction change mode in the first embodiment. In the example illustrated in FIG. 6, the support direction of the top plate 24 before the input for the support direction change mode is received is on the right side of the base 21 when the base 21 is viewed from the front, and the top plate support 231 supports the top plate main body 241 of the top plate 24 from below.

On the other hand, upon receiving the input for the support direction change mode in step S11 (step S11: Yes), the control function 411 determines whether the top plate support arm 23 supports the top plate (step S15). Specifically, the control function 411 determines whether the top plate support arm 23 supports the top plate based on a detection result of a sensor (not illustrated) or an input operation from the user regarding whether the top plate 24 is required on the top plate support arm 23.

Then, in step S15, when the top plate support arm 23 supports the top plate 24 (step S15: Yes), the control function 411 notifies the user (step S17). Specifically, the control function 411 notifies the user of information regarding detachment of the top plate 24 via the display 46. Then, the processing returns to step S13, and the processing from steps S13 to S17 is repeated until the control function 411 detects that the operator DO, who is a user, has detached the top plate 24 from the top plate support 231.

On the other hand, in step S15, when the top plate support arm 23 does not support the top plate 24 (step S15: No), the control function 411 moves the C-arm 25 to a predetermined position (step S19). Specifically, the control function 411 controls the C-arm support 222 via the power circuitry 29 to move the C-arm 25 to the vicinity of the base 21 as the predetermined position. Note that, when the C-arm 25 has already been moved to the predetermined position before execution of step S19, the processing of step S19 may be omitted.

Next, as illustrated in FIG. 5, the control function 411 rotates the main arm 221 (step S21). Specifically, the control function 411 causes the power circuitry 29 to rotate the main arm 221. More specifically, the control function 411 rotates the main arm 221 by 180° in the C direction illustrated in FIG. 2 about the first axis AX1 by the power circuitry 29. In this manner, the control function 411 moves the top plate support arm 23 in the left-to-right direction with respect to the base 21 by rotating the main arm 221 by 180° in the support direction change mode.

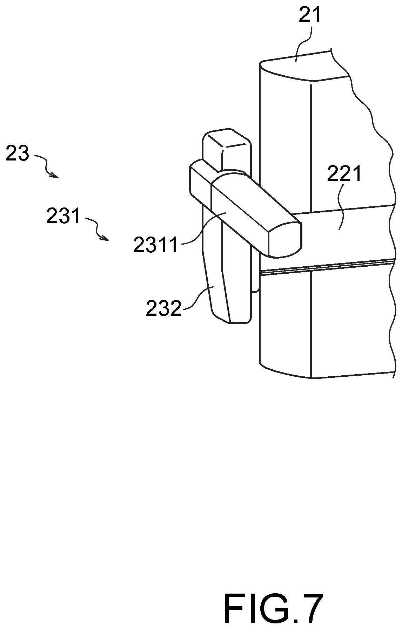

FIG. 7 is a diagram illustrating an example of the support direction of the top plate after the input for the support direction change mode is received in the first embodiment. As illustrated in FIG. 7, when the control function 411 receives the input for the support direction change mode and rotates the main arm 221, the support direction of the top plate support arm 23 is changed to the left side of the base 21 when the base 21 is viewed from the front. As illustrated in FIG. 7, the fixing mechanism 2312 is located below the top plate support main body 2311.

Next, as illustrated in FIG. 5, the control function 411 rotates the top plate support arm 23 (step S23). Specifically, the control function 411 controls the power circuitry 29 to rotate the top plate support 231 of the top plate support arm 23. More specifically, the control function 411 rotates the top plate support 231 by 180° in the F direction illustrated in FIG. 2 about the second axis AX2.

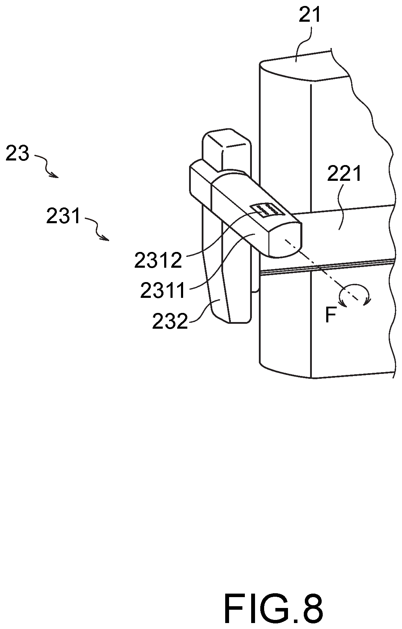

FIG. 8 is a diagram illustrating an example of a case in which the top plate support 231 in the top plate support arm 23 is rotated in the first embodiment. As illustrated in FIG. 8, the control function 411 rotates the top plate support 231 of the top plate support arm 23, so that the fixing mechanism 2312 of the top plate support 231 located on the lower side of the top plate support main body 2311 illustrated in FIG. 7 is located on the upper side of the top plate support main body 2311.

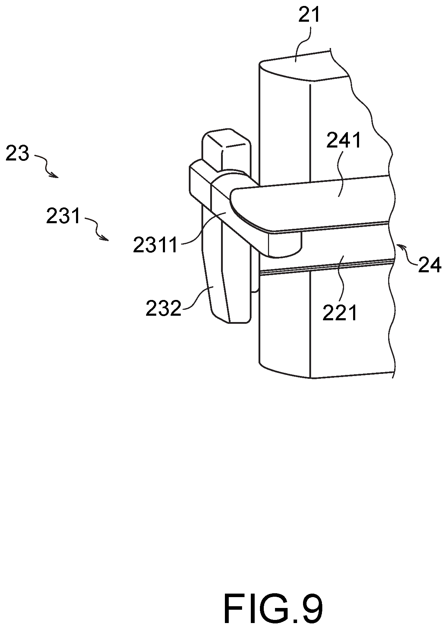

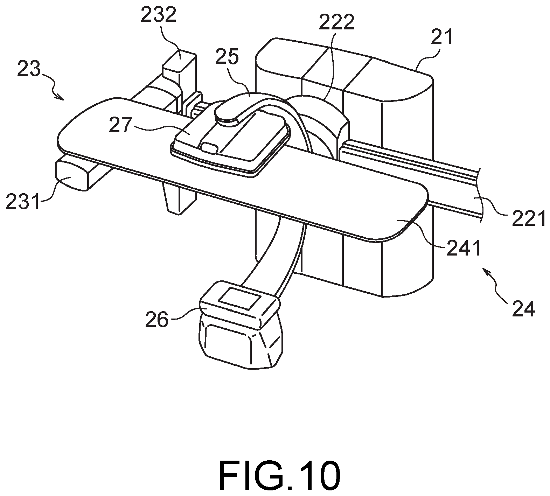

In step S23, the top plate support arm 23 is rotated to end the support direction change processing. Thereafter, the operator DO attaches the top plate 24 to the top plate support 231. FIG. 9 is a diagram illustrating an example of the top plate support 231 that supports the top plate 24 in the first embodiment. FIG. 10 is a diagram illustrating an example of the support direction of the top plate 24 after the support direction change mode is executed in the first embodiment. As illustrated in FIG. 8, since the fixing mechanism 2312 is located on the upper side of the top plate support 231, in the example illustrated in FIGS. 9 and 10, when the attachment portion 242 of the top plate 24 is attached to the fixing mechanism 2312, the top plate support main body 2311 supports the top plate 24 from below the top plate 24. That is, the top plate support arm 23 is configured such that the control function 411 can support the top plate 24 from below by rotating the top plate support arm 23 about the second axis AX2, which is the axis in the lateral direction of the top plate 24, before and after the support direction change mode is executed. As illustrated in FIG. 10, when the support direction change mode is executed, the support direction of the top plate 24 becomes the left side of the base 21 when the base 21 is viewed from the front.

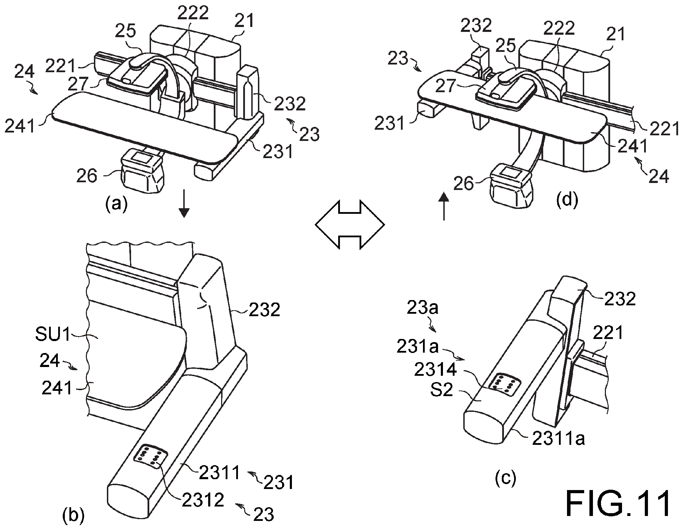

FIG. 11 is a diagram illustrating the X-ray diagnosis apparatus 1 before and after the support direction of the top plate 24 is changed. As illustrated in FIG. 11A, before the support direction change mode is executed, that is, before the support direction is changed, the support direction of the top plate 24 is on the right side of the base 21 when the base 21 is viewed from the front. Next, as illustrated in FIG. 11B, the operator detaches the top plate 24 from the top plate support arm 23 and then executes the support direction change mode, so that the control function 411 rotates the main arm 221 by 180°. Next, as illustrated in FIG. 11C, after the control function 411 rotates the top plate support arm 23 by 180°, the operator attaches the top plate 24 to the top plate support arm 23. As a result, as illustrated in FIG. 11D, after the support direction change mode is executed, that is, after the support direction is changed, the support direction of the top plate 24 is on the left side of the base 21 when the base 21 is viewed from the front from the attachment side of the C-arm 25. For this reason, in a procedure such as ERCP where an operator approaches from the left-hand side of the subject P, accessibility to the subject P is improved, and the workability of clinical procedures can be improved.

As described above, in the X-ray diagnosis apparatus 1 according to the first embodiment, by controlling the main arm 221 and the top plate support arm 23, the main arm 221 is rotated, and the top plate support 231 of the top plate support arm 23 is rotated, so that the user can arrange the top plate 24 above the top plate support 231 even when changing the support direction of the top plate 24. As a result, the support state of the top plate 24 can be changed according to the procedure.

As a comparative example of the X-ray diagnosis apparatus 1 according to the first embodiment, there is a technique in which an X-ray TV apparatus including a C-arm includes an X-ray TV mechanism capable of switching so that a top plate support arm can be arranged on each of the left and right sides when a main arm is rotated by 180° and the X-ray TV apparatus is viewed from the front, and both the front and back surfaces of the top plate can be used when the top plate support arm is switched so as to be arranged on each of the left and right sides. In such a technique, when the support direction is changed so that the top plate can be supported from the left side in a type in which the top plate is supported from the right side by rotating the main arm by 180°, or when the support direction is changed so that the top plate can be supported from the right side in a type in which the top plate is supported from the left side, the top plate support arm supports the top plate from above. Therefore, the length dimension of the top plate on which the subject can be placed is shortened by the top plate support arm, and the top plate support arm protrudes onto the top plate. Therefore, there is a problem in that the usability of the X-ray diagnosis apparatus including the C-arm deteriorates depending on the procedure.

On the other hand, according to the X-ray diagnosis apparatus 1 of the first embodiment, since the user can arrange the top plate 24 above the top plate support 231 even when the user changes the support direction of the top plate 24, the usability of the X-ray diagnosis apparatus 1 can be improved.

In the X-ray diagnosis apparatus 1 according to the first embodiment, the main arm 221 is rotated, and the top plate support 231 of the top plate support arm 23 is rotated, so that the main arm 221 and the top plate support arm 23 are controlled to change the support direction of the top plate 24. Therefore, it is not necessary to manufacture the X-ray diagnosis apparatus of the type selected by the user by selecting one of the two types of the type in which the top plate is supported from the right side and the type in which the top plate is supported from the left side before installation of the X-ray diagnosis apparatus Accordingly, it is possible to produce one type of X-ray diagnosis apparatus, which can contribute to improvement of productivity.

Note that, in the X-ray diagnosis apparatus 1 according to the first embodiment, the operator may manually rotate the top plate support arm 23. In this case, in step S21 of the support direction change processing illustrated in FIG. 5, before or after the control function 411 rotates the main arm 221 by controlling the main arm 221, the operator manually rotates the top plate support arm 23. Note that, when the X-ray diagnosis apparatus 1 is configured such that the operator manually rotates the top plate support arm 23, a lock pin that locks the rotation of the top plate support arm 23 is provided, the lock pin unlocks the rotation of the top plate support arm 23 before the top plate support arm 23 is manually rotated, and the lock pin locks the rotation of the top plate support arm 23 after the top plate support arm 23 is manually rotated. Note that the mechanism for locking the rotation of the top plate support arm 23 is not limited to the lock pin, and the configuration of the mechanism for locking the rotation is arbitrary. When the top plate support arm 23 is manually rotated, the processing of step S23 of the support direction change processing illustrated in FIG. 5 is omitted.

### [First Modification]

The X-ray diagnosis apparatus 1 according to the first embodiment may include a top plate detection sensor, which is a sensor for detecting the top plate 24.

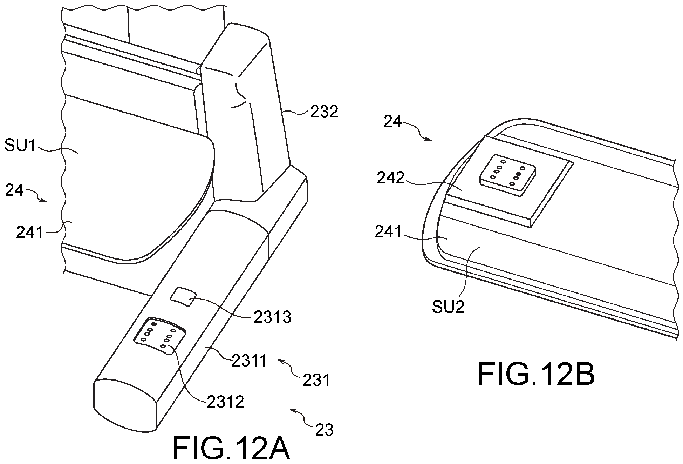

FIG. 12 is a diagram illustrating an example of a detailed configuration of a top plate support arm and a top plate according to a first modification, and is a diagram corresponding to FIG. 3. FIG. 12A is a diagram illustrating an example of a detailed configuration of the top plate support arm 23 according to the first modification, and FIG. 12B is a diagram illustrating an example of a detailed configuration of the top plate 24 according to the first modification. As illustrated in FIG. 12, the X-ray diagnosis apparatus 1 according to the present embodiment is configured by adding a top plate detection sensor 2313 to the X-ray diagnosis apparatus 1 according to the above-described first embodiment. Configurations and functions other than the top plate detection sensor 2313 are similar to those in FIG. 3 in the above-described first embodiment, and thus the description thereof will be omitted.

The top plate detection sensor 2313 is a sensor for detecting the top plate 24. The top plate detection sensor 2313 outputs a detection result of the top plate 24 to the processing circuitry 41. As illustrated in FIG. 12A, the top plate detection sensor 2313 is provided in the vicinity of the fixing mechanism 2312. The top plate detection sensor 2313 is, for example, a camera, an optical sensor, a weight sensor, a micro switch, or the like. The top plate detection sensor 2313 may be at least one of a camera, an optical sensor, and a weight sensor, or may include two or three of them.

By including the top plate detection sensor 2313, in step S15 of the support direction change processing executed in the X-ray diagnosis apparatus 1 according to the first embodiment, the control function 411 determines whether the top plate support arm 23 supports the top plate based on the detection result of the top plate 24 by the top plate detection sensor 2313.

Note that, in the above-described first modification, the control function 411 may determine whether the top plate support arm 23 supports the top plate 24, and may issue a notification when the top plate support arm 23 does not support the top plate 24. Specifically, after executing the support direction change mode, the control function 411 may determine whether the top plate support arm 23 supports the top plate 24 based on the detection result of a micro switch provided on the top plate support arm 23 as the top plate detection sensor 2313. When the top plate support arm 23 supports the top plate 24, the control function 411 enables the operation of the main arm 221 and the like to be performed by executing various imaging modes. Conversely, when the top plate support arm 23 does not support the top plate 24, the control function 411 may issue a notification indicating that the top plate support arm 23 does not support the top plate 24. As a result, in a state in which the top plate 24 is not attached to the top plate support arm 23, it is possible to prevent the main arm 221 from operating by the control function 411 executing various imaging modes.

Furthermore, in the above-described first modification, the control function 411 determines whether the top plate support arm 23 supports the top plate 24 and also determines whether the top plate 24 is fixed to the top plate support arm 23. When the top plate support arm 23 supports the top plate 24 and the top plate 24 is fixed to the top plate support arm 23, the control function 411 enables the operation of the main arm 221 and the like to be performed by executing various imaging modes. Conversely, when at least one of a case in which the top plate support arm 23 does not support the top plate 24 and a case in which the top plate 24 is not fixed to the top plate support arm 23 is satisfied, the control function 411 may issue a notification.

Specifically, the X-ray diagnosis apparatus 1 includes a top plate fixation detection sensor that detects that the top plate 24 is fixed to the top plate support arm 23 by the fixing mechanism 2312, and the top plate fixation detection sensor includes a camera, an optical sensor, a weight sensor, a micro switch, and the like. After executing the support direction change mode, the control function 411 determines whether the top plate support arm 23 supports the top plate 24 based on a detection result of the micro switch provided on the top plate support arm 23 as the top plate detection sensor 2313, and also determines whether the top plate 24 is fixed to the top plate support arm 23 based on a detection result of the micro switch provided on the top plate support arm 23 as the top plate fixation detection sensor. Then, when the top plate support arm 23 supports the top plate 24 and the top plate 24 is fixed to the top plate support arm 23, the control function 411 enables the operation of the main arm 221 and the like to be performed by executing various imaging modes. Conversely, when at least one of a case in which the top plate support arm 23 does not support the top plate 24 and a case in which the top plate 24 is not fixed to the top plate support arm 23 is satisfied, the control function 411 issues a notification indicating that there is an abnormality in at least one of a state in which the top plate support arm 23 does not support the top plate 24 and a state in which the top plate 24 is not fixed to the top plate support arm 23. As a result, in at least one of a state in which the top plate 24 is not attached to the top plate support arm 23 and a state in which the top plate 24 is not fixed to the top plate support arm 23, it is possible to reduce the possibility that the main arm 221 and the like operate due to the control function 411 executing various imaging modes.

### [Second Modification]

In the support direction change processing executed by the X-ray diagnosis apparatus 1 according to the above-described first embodiment, the top plate 24 is attached after the main arm 221 and the top plate support arm 23 are rotated. However, it is also possible that, after the top plate 24 is attached to the top plate support arm 23 so as to change the attachment orientation of the top plate 24, the support direction change mode is executed in a state in which the top plate 24, the attachment orientation of which has been changed, is supported, and the main arm 221 and the top plate support arm 23 are rotated. Note that, since the configuration and function of the X-ray diagnosis apparatus 1 are the same as those illustrated in FIGS. 1 and 2, the description thereof will be omitted.

Note that, although the configurations of the top plate support arm 23 and the top plate 24 according to a second modification are not illustrated, the top plate detection sensor is different from that in the above-described first modification, and thus the top plate detection sensor will be referred to as a top plate detection sensor 2313a. Note that configurations and functions other than the top plate detection sensor 2313a are the same as those in FIG. 12 of the above-described first modification, and thus the description thereof will be omitted.

The top plate detection sensor 2313a according to the present modification detects the top plate 24 and also detects the attachment orientation of the top plate 24. The top plate detection sensor 2313a outputs a detection result of the attachment orientation of the top plate 24 to the processing circuitry 41. The top plate detection sensor 2313a is provided in the vicinity of the fixing mechanism 2312. The top plate detection sensor 2313a according to the second modification is also, for example, a camera, an optical sensor, a weight sensor, or the like. Note that the top plate detection sensor 2313a may be at least one of a camera, an optical sensor, and a weight sensor, or may include two or three of them.

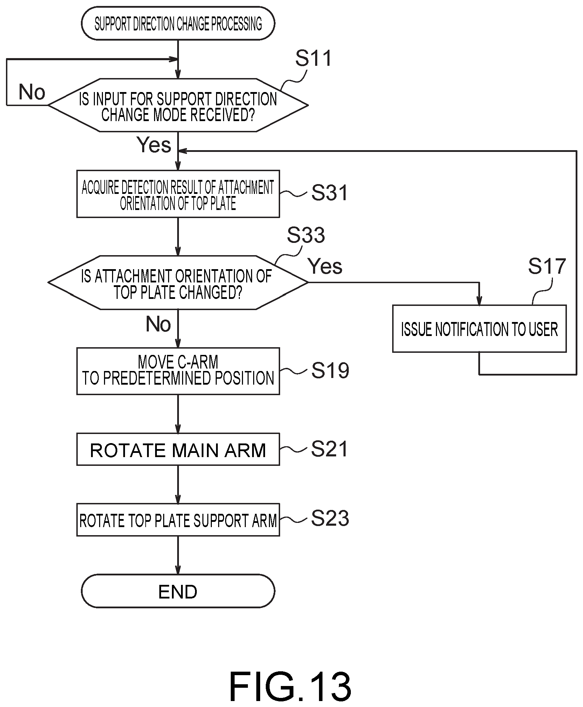

FIG. 13 is a flowchart for description of the support direction change processing executed in the X-ray diagnosis apparatus 1 according to the second modification, and is a diagram corresponding to FIG. 5. In the support direction change processing according to the second modification, the input for the support direction change mode is received, the detection result of the attachment orientation of the top plate 24 is acquired, the C-arm 25 is moved, the main arm 221 is rotated, and the top plate support arm 23 is rotated. For example, the support direction change processing is processing executed when the input for the support direction change mode is received. Note that the processing in step S11 illustrated in FIG. 13 is the same as that in FIG. 5, and thus the description thereof will be omitted.

In step S11, when the control function 411 receives the input for the support direction change mode (step S11: Yes), the control function 411 acquires the detection result of the attachment orientation of the top plate 24 (step S31). Specifically, the control function 411 acquires the detection result of the attachment orientation of the top plate 24 output from the top plate detection sensor 2313a.

Next, as illustrated in FIG. 13, the control function 411 determines whether the attachment orientation of the top plate 24 has been changed (step S33). Specifically, the control function 411 determines whether the attachment orientation of the top plate 24 has been changed based on the detection result of the top plate 24 acquired in step S31.

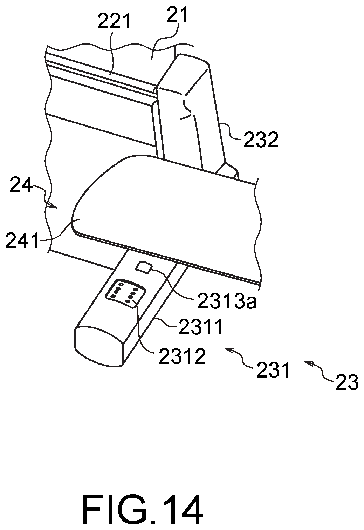

FIG. 14 is a diagram illustrating an example of a state in which the top plate 24, the attachment orientation of which has been changed, is attached to the top plate support arm 23 in the second modification. As illustrated in FIG. 14, an operator DO, who is a user, detaches the top plate 24 supported by the top plate support arm 23 in the attachment orientation illustrated in FIG. 12A from the top plate support arm 23, changes the attachment orientation of the top plate 24 so as to be axisymmetric about the second axis AX2 of the top plate support arm 23, and attaches the top plate 24, the attachment orientation of which has been changed, to the top plate support arm 23, thereby changing the attachment orientation of the top plate 24. Then, the control function 411 determines whether the top plate 24 is supported in the attachment orientation illustrated in FIG. 14.

Note that the processing in steps S17 to S23 after step S33 is equivalent to the above-described processing in steps S17 to S23 in FIG. 5, and thus the description thereof will be omitted. Then, in step S23, the top plate support arm 23 is rotated to end the support direction change processing according to the second modification.

As described above, in the X-ray diagnosis apparatus 1 according to the second modification, in the state in which the top plate 24, the attachment orientation of which has been changed, is attached to the top plate support arm 23, the main arm 221 and the top plate support arm 23 are controlled so as to rotate the main arm 221 and the top plate support 231 in the top plate support arm 23. That is, in the present modification, the control function 411 executes the support direction change mode in the state in which the top plate 24, the attachment orientation of which has been changed, is supported by the top plate support arm. Therefore, even when the support direction of the top plate 24 is changed in the state in which the top plate 24, the attachment orientation of which has been changed, is attached to the top plate support arm 23, the top plate 24 can be arranged above the top plate support 231, and the support state of the top plate 24 can also be changed according to the procedure.

In addition, the control function 411 according to the second modification executes the support direction change mode in the state in which the top plate 24, the attachment orientation of which has been changed, is supported by the top plate support arm, and thus, it is possible to reduce the work of detaching the top plate 24 and retracting the top plate 24 to a position not interfering with each part of the X-ray diagnosis apparatus 1 in the support direction change mode, thereby making it possible to reduce the number of work steps of the user.

### [Third Modification]

In the X-ray diagnosis apparatus 1 according to the first embodiment, after the support direction of the top plate 24 is changed, the height of the top plate 24 from the floor surface after the change of the support direction of the top plate 24 can be corrected such that the height of the top plate 24 from the floor surface before the change of the support direction of the top plate 24 coincides with the height of the top plate 24 from the floor surface after the change of the support direction of the top plate 24. Note that, since the configuration and function of the X-ray diagnosis apparatus 1 are the same as those illustrated in FIGS. 1 and 2, the description thereof will be omitted.

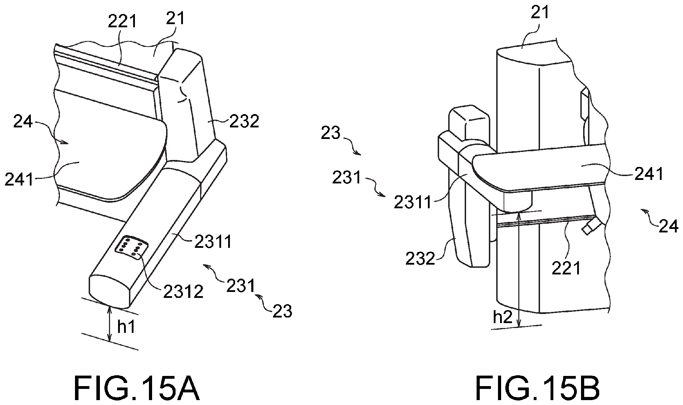

FIG. 15 is a diagram illustrating an example of the height of the top plate support arm 23 from the floor surface before and after the change of the support direction of the top plate 24 in a third modification. FIG. 15A is a diagram illustrating an example of the height of the top plate support arm 23 from the floor surface before the support direction of the top plate 24 is changed. FIG. 15B is a diagram illustrating an example of the height of the top plate support arm 23 from the floor surface after the support direction is changed. As illustrated in FIG. 15A, the height from the floor surface to the lower end of the top plate support arm 23 before the support direction is changed is h1. On the other hand, as illustrated in FIG. 15B, the height from the floor surface to the lower end of the top plate support arm 23 after the support direction of the top plate 24 is changed is h2.

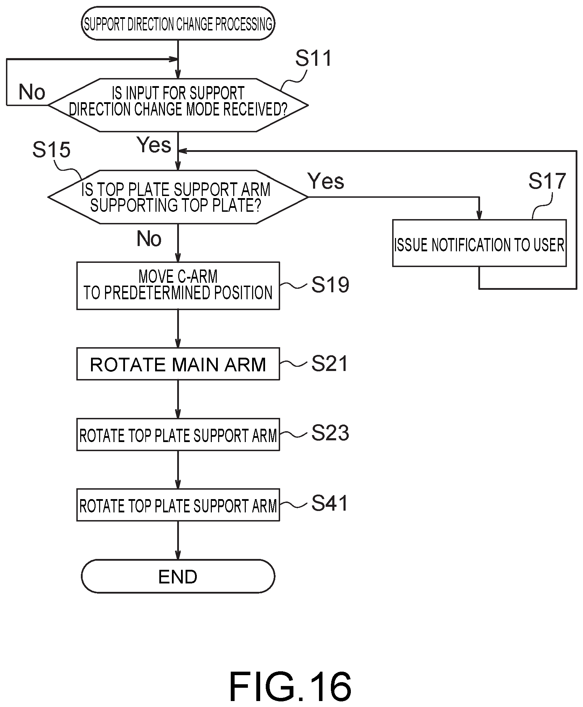

FIG. 16 is a flowchart for description of the support direction change processing executed in the X-ray diagnosis apparatus 1 according to the third modification, and is a diagram corresponding to FIG. 5. In the support direction change processing according to the third modification, the input for the support direction change mode is received, the C-arm 25 is moved, the main arm 221 is rotated, the top plate support arm 23 is rotated, and the height of the top plate 24 is corrected. For example, the support direction change processing is processing executed when the input for the support direction change mode is received. Note that the processing in steps S11 to S23 illustrated in FIG. 16 is the same as that in FIG. 5, and thus the description thereof will be omitted.

Next, as illustrated in FIG. 16, the control function 411 in the processing circuitry 41 of the X-ray diagnosis apparatus 1 corrects the height of the top plate 24 (step S41). Specifically, the control function 411 moves at least one of the main arm 221 and the top plate support arm 23 in the vertical direction to correct the height of the top plate 24, so that the heights of the top plate 24 before and after the execution of the support direction change mode substantially coincide with each other. More specifically, as illustrated in FIG. 15, when the height from the floor surface to the lower end of the top plate support arm 23 before the change of the support direction of the top plate 24 is h1, and the height from the floor surface to the lower end of the top plate support arm 23 after the change of the support direction of the top plate 24 is h2, the control function 411 controls, for example, at least one of the main arm 221 and the top plate support arm 23 to correct the height of the top plate 24 so that the height from the floor surface to the lower end of the top plate support arm 23 after the change of the support direction of the top plate 24 becomes h1.

As described above, also in the X-ray diagnosis apparatus 1 according to the third modification, the main arm 221 and the top plate support arm 23 are controlled so as to rotate the main arm 221 and the top plate support 231 of the top plate support arm 23. Accordingly, even when the support direction of the top plate 24 is changed in a state in which the top plate 24, the attachment orientation of which has been changed, is attached to the top plate support arm 23, the user can arrange the top plate 24 above the top plate support 231 and can also change the support state of the top plate 24 according to the procedure.

In the X-ray diagnosis apparatus 1 according to the third modification, the control function 411 controls the main arm 221 and/or the top plate support arm 23 to correct the height of the top plate 24. Accordingly, even if the height of the top plate 24 from the floor surface is changed by changing the support state of the top plate 24 according to the procedure, the height of the top plate 24 can be maintained.

### [Second Embodiment]

In the X-ray diagnosis apparatus 1 according to the above-described first embodiment, it has been described that the top plate support 231 is configured to be rotatable, and the fixing mechanism 2312 positioned below the top plate support 231 is moved upward by rotating the main arm 221, thereby making it possible to position the top plate 24 above the top plate support 231. However, the method of positioning the top plate 24 above the top plate support 231 is not limited thereto. In a second embodiment, the top plate support arm 23 in the X-ray diagnosis apparatus 1 may include a plurality of support surfaces that support the top plate 24 so that the top plate 24 is positioned above the top plate support 231.

Note that, although the configuration of the X-ray diagnosis apparatus according to the second embodiment is not illustrated, the top plate support arm 23 according to the present embodiment is different from that in the above-described first embodiment, and thus the top plate support arm 23 will be referred to as a top plate support arm 23a. Note that configurations and functions other than the top plate support arm 23a are the same as those of the first embodiment, and thus the description thereof will be omitted.

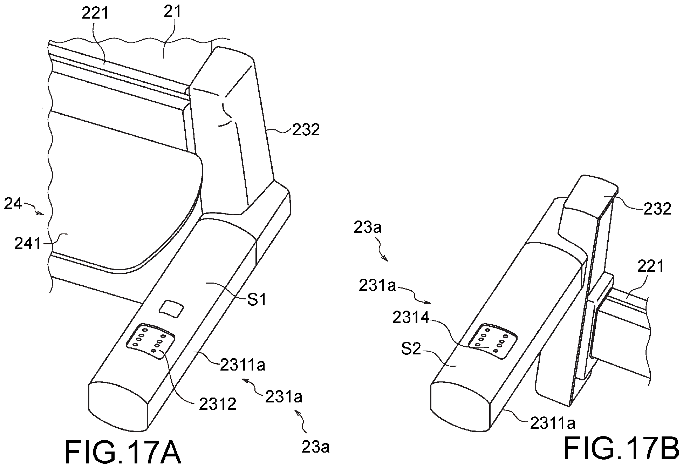

The top plate support arm 23a according to the second embodiment is connected to the main arm 221 and supports the top plate 24. The top plate support arm 23a is supported by the main arm 221 in a cantilevered (or double-supported) manner. The top plate support arm 23a can be slid in the E direction with respect to the main arm 221 by the power circuitry 29. As illustrated in FIG. 17, the top plate support arm 23a includes a top plate support 231a and the connecting portion 232. Note that, since the configuration of the connecting portion 232 is the same as that of the first embodiment, the description thereof will be omitted.

The top plate support 231a according to the present embodiment includes a plurality of support surfaces for supporting the top plate 24. Here, the support surface refers to a surface capable of supporting the top plate 24, among a plurality of surfaces obtained by dividing a surface of a top plate support main body 2311a in the top plate support 231a, which is parallel to the lateral direction of the top plate 24, into a plurality of surfaces along the lateral direction of the top plate 24.

FIG. 17 is a diagram illustrating an example of a detailed configuration of the top plate support arm 23a and the top plate 24 according to the second embodiment. FIG. 17A is a diagram illustrating an example of a case in which the support direction of the top plate 24 according to the second embodiment is on the right side of the base 21 when the base 21 is viewed from the front, and FIG. 17B is a diagram illustrating an example of a case in which the support direction of the top plate 24 according to the second embodiment is on the left side of the base 21 when the base 21 is viewed from the front. As illustrated in FIG. 17, the top plate support main body 2311a according to the present embodiment includes a first support surface S1 capable of supporting the top plate 24 by being equipped with a first fixing mechanism 2312, and a second support surface S2 capable of supporting the top plate 24 by being equipped with a second fixing mechanism 2314, among first to fourth support surfaces S1 to S4, which are surfaces obtained by dividing the top plate support main body 2311a parallel to the lateral direction of the top plate 24 into four surfaces along the lateral direction of the top plate 24. Accordingly, a top plate support 231a according to the present embodiment includes two support surfaces. That is, the top plate support main body 2311a of the top plate support 231a in the top plate support arm 23a includes, as a plurality of surfaces, the first support surface S1 of the top plate support main body 2311a and the second support surface S2 of the top plate support main body 2311a in the top plate support arm 23a. The plurality of support surfaces, which are the first support surface S1 and the second support surface S2, are provided symmetrically in a direction orthogonal to the longitudinal direction and the lateral direction of the top plate 24.

The top plate support 231a includes the first fixing mechanism 2312 and the second fixing mechanism 2314. The first fixing mechanism 2312 and the second fixing mechanism 2314 are mechanisms for fixing the top plate 24. The specific configurations of the first fixing mechanism 2312 and the second fixing mechanism 2314 are the same as those of the fixing mechanism 2312 described above, and thus the description thereof will be omitted.

The top plate support 231a according to the present embodiment does not rotate in the lateral direction of the top plate 24 with respect to the connecting portion 232. Therefore, the control function 411 according to the present embodiment controls the main arm 221, the C-arm support 222, the C-arm 25, the X-ray tube 26, and the X-ray detector 27 by controlling the high-voltage power supply 28 and the power circuitry 29. Specifically, the control function 411 controls the main arm 221 to rotate the main arm 221. Note that the functions of the control function 411 according to the second embodiment other than those described above are equivalent to those of the first embodiment, and thus the description thereof will be omitted.

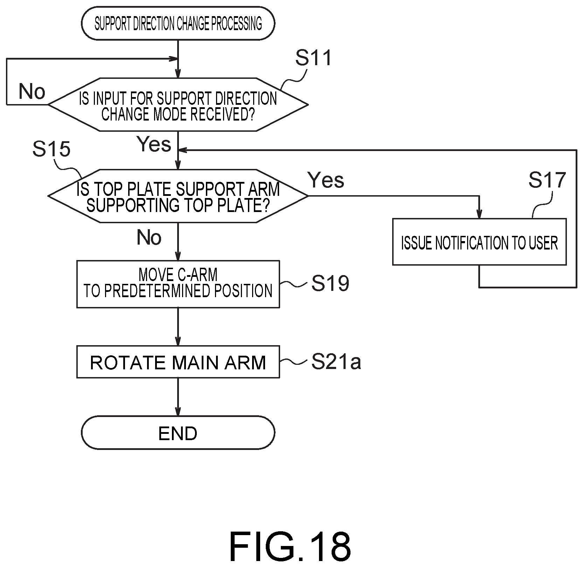

FIG. 18 is a flowchart for description of support direction change processing executed in the X-ray diagnosis apparatus 1 according to the second embodiment. In this support direction change processing, the input for the support direction change mode is received, the C-arm 25 is moved, and the main arm 221 is rotated. For example, the support direction change processing is processing executed when the input for the support direction change mode is received. Note that the processing in steps S11 to S19 illustrated in FIG. 18 is the same as that in FIG. 5, and thus the description thereof will be omitted.

Next, as illustrated in FIG. 18, the control function 411 in the processing circuitry 41 of the X-ray diagnosis apparatus 1 rotates the main arm 221 (step S21a). Specifically, the control function 411 controls the power circuitry 29 to rotate the main arm 221. More specifically, the control function 411 controls the power circuitry 29 to rotate the main arm 221 by 180° in the C direction illustrated in FIG. 2 about the first axis AX1. When the control function 411 rotates the main arm 221 by 180°, as illustrated in FIG. 17B, the first fixing mechanism 2312 is positioned on the lower side of the top plate support 231a, and the second fixing mechanism 2314 is positioned on the upper side of the top plate support 231a. In the support direction change mode, the control function 411 rotates the main arm 221 by 180° to move the top plate support arm 23a in the left-to-right direction with respect to the base 21.

In step S21a, the top plate support arm 23a is rotated to end the support direction change processing according to the second embodiment. Thereafter, an operator DO, who is a user, attaches the top plate 24 to the top plate support 231a. When the operator DO, who is a user, attaches the attachment portion 242 of the top plate 24 to the fixing mechanism, the top plate support 231a supports the top plate 24 from below the top plate 24. By executing the support direction change mode, the support direction of the top plate 24 becomes the left side of the base 21 when the base 21 is viewed from the front.

As described above, in the X-ray diagnosis apparatus 1 according to the second embodiment, the top plate support arm 23a includes a plurality of surfaces for supporting the top plate, and the control function 411 controls the main arm 221 so as to rotate the main arm 221. Therefore, the user can arrange the top plate 24 above the top plate support 231a even when changing the support direction of the top plate 24, and can also change the support state of the top plate 24 according to the procedure.

### [Fourth Modification]

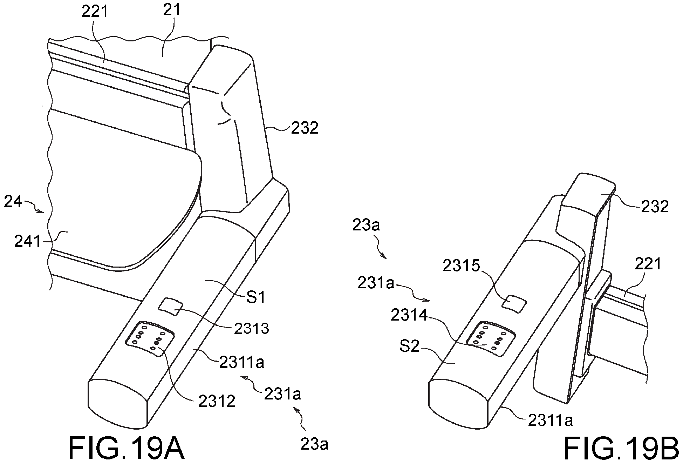

The X-ray diagnosis apparatus 1 according to the above-described second embodiment may include a plurality of top plate detection sensors that are sensors for detecting the top plate 24. FIG. 19 is a diagram illustrating an example of a detailed configuration of the top plate support arm 23a and the top plate 24 according to a fourth modification. FIG. 19A is a diagram illustrating an example of a detailed configuration of the top plate support arm 23a according to the fourth modification, and FIG. 19B is a diagram illustrating an example of a detailed configuration of the top plate 24 according to the fourth modification. The top plate support 231a according to the fourth modification includes a first top plate detection sensor 2313 and a second top plate detection sensor 2315. The first top plate detection sensor 2313 and the second top plate detection sensor 2315 are sensors for detecting the top plate 24.

Specifically, the first top plate detection sensor 2313 detects the top plate 24 supported by the first support surface S1, that is, fixed to the first fixing mechanism 2312. Therefore, as illustrated in FIG. 19A, the first top plate detection sensor 2313 is provided in the vicinity of the first fixing mechanism 2312. The first top plate detection sensor 2313 outputs a detection result of the top plate 24 fixed to the first fixing mechanism 2312 to the processing circuitry 41.

The second top plate detection sensor 2315 detects the top plate 24 supported by the second support surface S2, that is, fixed to the second fixing mechanism 2314. Therefore, as illustrated in FIG. 19B, the second top plate detection sensor 2315 is provided in the vicinity of the second fixing mechanism 2314. The second top plate detection sensor 2315 outputs a detection result of the top plate 24 fixed to the second fixing mechanism 2314 to the processing circuitry 41.

The first top plate detection sensor 2313 and the second top plate detection sensor 2315 are, for example, a camera, an optical sensor, a weight sensor, a micro switch, and the like. Note that the top plate support 231a may include one camera as the first top plate detection sensor 2313 and the second top plate detection sensor 2315.

By providing the first top plate detection sensor 2313 and the second top plate detection sensor 2315, in step S15 of the support direction change processing executed in the X-ray diagnosis apparatus 1 according to the above-described second embodiment, the control function 411 determines whether the top plate support arm 23a supports the top plate 24 based on the detection result of the top plate 24 by the first top plate detection sensor 2313 or the second top plate detection sensor 2315.

Note that, in the above-described fourth modification, the control function 411 may determine whether the top plate support arm 23a supports the top plate 24, and may issue a notification when the top plate support arm 23a does not support the top plate 24. Specifically, after executing the support direction change mode, the control function 411 may determine whether the top plate support arm 23a supports the top plate 24 based on the detection results of the micro switches provided on the top plate support arm 23a as the first top plate detection sensor 2313 and the second top plate detection sensor 2315. When the top plate support arm 23a supports the top plate 24, the control function 411 enables the operation of the main arm 221 and the like to be performed by executing various imaging modes. Conversely, when the top plate support arm 23a does not support the top plate 24, the control function 411 may issue a notification indicating that the top plate support arm 23a does not support the top plate 24. More specifically, the control function 411 acquires information regarding the support direction of the top plate 24 after the support direction change mode, and determines whether the top plate support arm 23a supports the top plate 24 in a state in which the first top plate surface SU1 of the top plate 24 faces upward, based on the information regarding the support direction of the top plate 24 and the detection results of the micro switches provided on the top plate support arm 23a as the first top plate detection sensor 2313 and the second top plate detection sensor 2315. When the top plate support arm 23a supports the top plate 24 in a state in which the first top plate surface SU1 of the top plate 24 faces upward, the control function 411 enables the operation of the main arm 221 and the like to be performed by executing various imaging modes. Conversely, in a state in which the first top plate surface SU1 of the top plate 24 faces upward, when the top plate support arm 23a does not support the top plate 24, the control function 411 may issue a notification indicating that the top plate support arm 23a does not support the top plate 24. As a result, in a state in which the top plate 24 is not attached to the top plate support arm 23a, it is possible to reduce the possibility that the main arm 221 and the like operate due to the control function 411 executing various imaging modes.

Furthermore, in the above-described fourth modification, the control function 411 determines whether the top plate support arm 23a supports the top plate 24 and also determines whether the top plate 24 is fixed to the top plate support arm 23a. When the top plate support arm 23a supports the top plate 24 and the top plate 24 is fixed to the top plate support arm 23a, the control function 411 enables the operation of the main arm 221 and the like to be performed by executing various imaging modes. Conversely, when at least one of a case in which the top plate support arm 23a does not support the top plate 24 and a case in which the top plate 24 is not fixed to the top plate support arm 23a is satisfied, the control function 411 may issue a notification.

Specifically, the X-ray diagnosis apparatus 1 includes a first top plate fixation detection sensor including a camera, an optical sensor, a weight sensor, a micro switch, and the like, which detects that the top plate 24 is fixed to the top plate support arm 23a by the first fixing mechanism 2312, and a second top plate fixation detection sensor including a camera, an optical sensor, a weight sensor, a micro switch, and the like, which detects that the top plate 24 is fixed to the top plate support arm 23a by the second fixing mechanism 2314. After executing the support direction change mode, the control function 411 determines whether the top plate support arm 23a supports the top plate 24 based on detection results of the micro switches provided on the top plate support arm 23a as the first top plate detection sensor 2313 and the second top plate detection sensor 2315, and also determines whether the top plate 24 is fixed to the top plate support arm 23a based on detection results of micro switches provided on the top plate support arm 23a as the first top plate fixation detection sensor and the second top plate fixation detection sensor. Then, when the top plate support arm 23a supports the top plate 24 and the top plate 24 is fixed to the top plate support arm 23a, the control function 411 enables the operation of the main arm 221 and the like to be performed by executing various imaging modes. Conversely, when at least one of a case in which the top plate support arm 23a does not support the top plate 24 and a case in which the top plate 24 is not fixed to the top plate support arm 23a is satisfied, the control function 411 issues a notification indicating that there is an abnormality in at least one of a state in which the top plate support arm 23a does not support the top plate 24 and a state in which the top plate 24 is not fixed to the top plate support arm 23a.

More specifically, after executing the support direction change mode, the control function 411 acquires information regarding the support direction of the top plate 24 after the support direction change mode, determines whether the top plate support arm 23a supports the top plate 24 in a state in which the first top plate surface SU1 of the top plate 24 faces upward based on the information regarding the support direction of the top plate 24 and the detection results of the micro switches provided on the top plate support arm 23a as the first top plate detection sensor 2313 and the second top plate detection sensor 2315, and also determines whether the top plate 24 is fixed to the top plate support arm 23a in the state in which the first top plate surface SU1 of the top plate 24 faces upward based on the detection results of the micro switches provided on the top plate support arm 23a as the first top plate fixation detection sensor and the second top plate fixation detection sensor, Then, when the top plate support arm 23a supports the top plate 24 and the top plate 24 is fixed to the top plate support arm 23a, the control function 411 enables the operation of the main arm 221 and the like to be performed by executing various imaging modes. Conversely, when at least one of a case in which the top plate support arm 23a does not support the top plate 24 and a case in which the top plate 24 is not fixed to the top plate support arm 23a is satisfied, the control function 411 issues a notification indicating that there is an abnormality in at least one of a state in which the top plate support arm 23a does not support the top plate 24 and a state in which the top plate 24 is not fixed to the top plate support arm 23a in the state in which the first top plate surface SU1 of the top plate 24 faces upward. As a result, in at least one of a state in which the top plate 24 is not attached to the top plate support arm 23a and a state in which the top plate 24 is not fixed to the top plate support arm 23a, it is possible to reduce the possibility that the main arm 221 and the like operate due to the control function 411 executing various imaging modes.

### [Third Embodiment]

In the X-ray diagnosis apparatus 1 according to the above-described second embodiment and fourth modification, each of the first fixing mechanism 2312 and the second fixing mechanism 2314 includes the locking mechanism in order to detachably fix the top plate 24 to the top plate support 231a. However, detachably fixing the top plate 24 to the top plate support 231a is not limited to a case in which each of the first fixing mechanism 2312 and the second fixing mechanism 2314 includes the locking mechanism. In the third embodiment, one fixer may be provided for detachably fixing the top plate 24 to the top plate support 231a.

Note that, although the configuration of the X-ray diagnosis apparatus according to the third embodiment is not illustrated, since the top plate support arm 23a and the top plate 24 according to the present embodiment are different from those of the above-described second embodiment, the top plate support arm 23a and the top plate 24 will be referred to as a top plate support arm 23b and a top plate 24a. Note that configurations and functions other than the top plate support arm 23b and the top plate 24a are the same as those of the second embodiment, and thus the description thereof will be omitted.

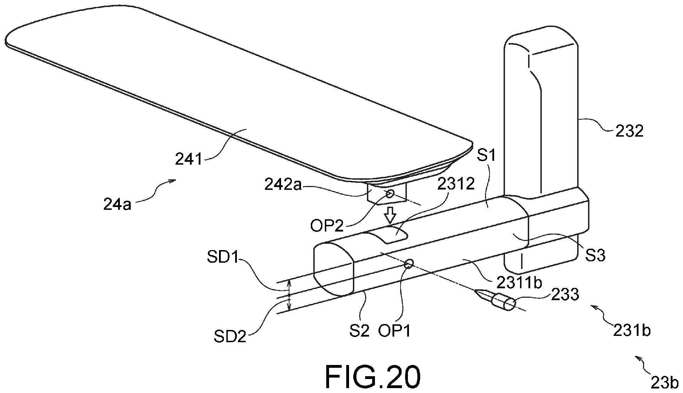

FIG. 20 is a diagram illustrating an example of a detailed configuration of the top plate support arm 23b and the top plate 24a according to the third embodiment. As illustrated in FIG. 20, the top plate support arm 23b according to the third embodiment includes a top plate support 231b, the connecting portion 232, and a fixer 233. The top plate 24a includes the top plate main body 241 and an attachment portion 242a. Note that, since the configuration of the connecting portion 232 is the same as that of the above-described first embodiment, the description thereof will be omitted. Further, since the configuration of the top plate main body 241 is the same as that of the above-described first embodiment, the description thereof will be omitted.

A second insertion hole OP2 formed for the fixer 233 to be inserted thereinto is formed in the attachment portion 242a according to the present embodiment.

As in the second embodiment, the top plate support 231b according to the present embodiment includes a plurality of support surfaces that support the top plate. Specifically, as illustrated in FIG. 20, since a top plate support main body 2311b of the top plate support 231b includes the first support surface S1 that includes the first fixing mechanism 2312 to support the top plate 24a and the second support surface S2 that includes the second fixing mechanism 2314 to support the top plate 24a, the top plate support 231b according to the present embodiment includes two support surfaces. The first support surface S1 and the second support surface S2, which are a plurality of surfaces, are provided symmetrically in a direction orthogonal to the longitudinal direction and the lateral direction of the top plate 24a. In addition, in the top plate support main body 2311b of the top plate support 231b, a first insertion hole OP1 is formed for inserting the fixer 233 that fixes the top plate 24a supported by the first support surface S1 when the top plate 24a is supported by the first support surface S1, and fixes the top plate 24a supported by the second support surface S2 when the top plate 24a is supported by the second support surface S2, thereby detachably fixing the top plate 24a. In the third embodiment, in each of a case in which the top plate 24a is supported by the first support surface S1 and a case in which the top plate 24a is supported by the second support surface S2, a distance SD1 from the upper end of a third surface S3 to the first insertion hole OP1 is equal to a distance SD2 from the lower end of the third surface S3 to the first insertion hole OP1 such that the top plate 24a can be fixed by the fixer 233 inserted into the first insertion hole OP1. Note that the first insertion hole OP1 corresponds to an insertion hole in the present embodiment.

The fixer 233 is a lock pin.

In the present embodiment, when the top plate 24a is attached to the first fixing mechanism 2312 of the top plate support 231b, the fixer 233 is inserted into the first insertion hole OP1 formed in the top plate support main body 2311b of the top plate support 231b and the second insertion hole OP2 in the attachment portion 242a of the top plate 24a attached to the first fixing mechanism 2312 of the top plate support 231b, whereby the top plate main body 241 is fixed to the top plate support 231b. When the top plate 24a is attached to the first fixing mechanism 2312 of the top plate support 231b, the fixation of the top plate main body 241 to the top plate support 231b is released by detaching the fixer 233 from the first insertion hole OP1 formed in the top plate support 231b and the second insertion hole OP2 of the attachment portion 242a of the top plate 24a attached to the first fixing mechanism 2312 of the top plate support 231b.

When the top plate 24a is attached to the second fixing mechanism 2314 of the top plate support 231b, the fixer 233 is inserted into the first insertion hole OP1 formed in the top plate support main body 2311b of the top plate support 231b and the second insertion hole OP2 of the attachment portion 242a of the top plate 24a attached to the second fixing mechanism 2314 of the top plate support 231b, whereby the top plate main body 241 is fixed to the top plate support 231b. When the top plate 24a is attached to the second fixing mechanism 2314 of the top plate support 231b, the fixation of the top plate main body 241 to the top plate support 231b is released by detaching the fixing mechanism 233 from the first insertion hole OP1 formed in the top plate support 231b and the second insertion hole OP2 of the attachment portion 242a of the top plate 24a attached to the second fixing mechanism 2314 of the top plate support 231b. That is, in the case of changing the support direction, in both a case in which the attachment portion 242a of the top plate 24a is attached to the first fixing mechanism 2312 and a case in which the attachment portion 242a of the top plate 24a is attached to the second fixing mechanism 2314, the top plate 24a can be fixed to the top plate support arm 23b by inserting the fixer 233 into the first insertion hole OP1 of the top plate support 231b and the second insertion hole OP2 of the attachment portion 242a, and the fixation can be released by detaching the fixer 233 from the first insertion hole OP1 of the top plate support 231b and the second insertion hole OP2 of the attachment portion 242a.

As described above, in the X-ray diagnosis apparatus 1 according to the third embodiment, the first insertion hole OP1 is formed at a position where the distance SD1 from the upper end of the third surface S3 to the first insertion hole OP1 is equal to the distance SD2 from the lower end of the third surface S3 to the first insertion hole OP1. Accordingly, when the support direction is changed, in both a case in which the attachment portion 242a of the top plate 24a is attached to the first fixing mechanism 2312 and a case in which the attachment portion 242a of the top plate 24a is attached to the second fixing mechanism 2314, the top plate 24a can be fixed to the top plate support arm 23b by inserting the fixer 233 into the first insertion hole OP1 of the top plate support 231b and the second insertion hole OP2 of the attachment portion 242a, or the fixation can be released by detaching the fixer 233 from the first insertion hole OP1 of the top plate support 231b and the second insertion hole OP2 of the attachment portion 242a. Consequently, without providing an insertion hole or a locking mechanism corresponding to each of the first fixing mechanism 2312 and the second fixing mechanism 2314, it is possible to simplify the configuration of the top plate support arm 23b and to detachably fix the top plate 24a attached to each of the first fixing mechanism 2312 and the second fixing mechanism 2314 to the top plate support arm 23b.

### [Fifth Modification]

In the X-ray diagnosis apparatus 1 according to the above-described third embodiment, when at least one of a case in which a state in which the fixation lock of the top plate 24a is released is detected and a case in which a state in which the top plate 24a is not attached to the top plate support arm 23b is detected is satisfied, the control function 411 can restrict the operation of the main arm 221 and the like. Note that, although the configuration of the X-ray diagnosis apparatus 1 according to the fifth modification is not illustrated, the X-ray diagnosis apparatus 1 is configured by adding a locked state detection sensor and a top plate detection sensor. Note that, since the top plate detection sensor according to the first modification is equivalent to the top plate detection sensor 2313 according to the above-described first modification, the description thereof will be omitted. The locked state detection sensor is substantially equivalent to the above-described top plate fixation detection sensor.

The locked state detection sensor is a sensor that detects a locked state in which the top plate 24a is fixed by a lock pin, which is the fixer 233. The locked state detection sensor is provided in the vicinity of the second insertion hole OP2 into which the lock pin is inserted. The locked state detection sensor is, for example, a camera, an optical sensor, a micro switch, or the like.

The control function 411 according to the fifth modification restricts the operation of the main arm 221 when at least one of a case in which the locked state detection sensor detects a state in which the fixation lock of the top plate 24a is released and a case in which the top plate detection sensor detects a state in which the top plate 24a is not attached is satisfied.

As described above, the X-ray diagnosis apparatus 1 according to the fifth modification includes the locked state detection sensor and the top plate detection sensor, and the control function 411 restricts the operation of the main arm 221 when the locked state detection sensor detects the state in which the fixation lock of the top plate 24a is released or when the top plate detection sensor satisfies at least one of the state in which the top plate 24a is not attached, so that the safety during the operation of the main arm can be improved.

Note that, in the fifth modification, when at least one of a case in which the locked state detection sensor detects a state in which the fixation lock of the top plate 24a is released and a case in which the top plate detection sensor detects a state in which the top plate 24a is not attached is satisfied, the control function 411 may issue a notification instead of restricting the operation of the main arm 221. As a result, in at least one of a state in which the top plate 24a is not attached to the top plate support arm 23b and a state in which the top plate 24a is not fixed to the top plate support arm 23b, it is possible to reduce the possibility that the main arm 221 and the like operate due to the control function 411 executing various imaging modes.

### [Fourth Embodiment]

In the X-ray diagnosis apparatus 1 according to the above-described first embodiment, the support direction of the top plate 24 is changed in the state in which the top plate 24 is detached, but the change of the support direction of the top plate 24 is not limited to the case in which the top plate 24 is detached. In a fourth embodiment, the control function may change the support direction of the top plate 24 without detachment of the top plate 24 by operating the main arm 221 and the top plate support arm 23 in a composite manner.

Note that, although the configuration of the X-ray diagnosis apparatus according to the fourth embodiment is not illustrated, the control function in the processing circuitry 41 of the image processing apparatus 12 according to the present embodiment is different from that of the first embodiment, and thus the control function will be referred to as a control function 411a. Note that configurations and functions other than the control function 411a are the same as those of the first embodiment, and thus the description thereof will be omitted.

The control function 411a according to the fourth embodiment controls the main arm 221, the C-arm support 222, the top plate support arm 23, the C-arm 25, the X-ray tube 26, and the X-ray detector 27 by controlling the high-voltage power supply 28 and the power circuitry 29. Specifically, the control function 411a controls the main arm 221 and the top plate support arm 23 to rotate the main arm 221 and rotate the top plate support arm 23 about the axis in the lateral direction of the top plate 24. In the present embodiment, the control function 411a executes the support direction change mode in a state in which the top plate 24 is supported by the top plate support arm 23. Here, the support direction change mode is a mode for changing the support direction of the top plate 24. The support direction change mode is an example of a first control mode in the fourth embodiment. Note that the configurations of the top plate support arm 23 and the top plate 24 according to the fourth embodiment are the same as those of the above-described first embodiment, and thus the description thereof will be omitted.

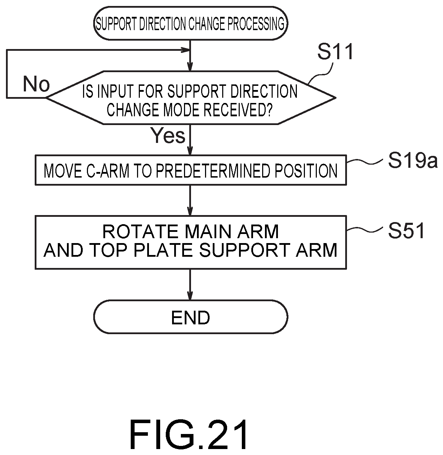

FIG. 21 is a flowchart for description of support direction change processing executed in the X-ray diagnosis apparatus 1 according to the fourth embodiment, and is a diagram corresponding to FIG. 5. In the support direction change processing according to the fourth embodiment, the input for the support direction change mode is received, the C-arm 25 is moved, and the C-arm 25, the main arm 221, and the top plate support arm 23 are rotated. For example, the support direction change processing is processing executed when the input for the support direction change mode is received. Note that the processing in step S11 illustrated in FIG. 21 is the same as that in FIG. 5, and thus the description thereof will be omitted.

Upon receiving the input for the support direction change mode in step S11 (step S11: Yes), the control function 411a in the processing circuitry 41 of the image processing apparatus 12 moves the C-arm 25 to a predetermined position (step S19a). Specifically, the control function 411a controls the C-arm support 222 via the power circuitry 29 to move the C-arm support 222 to the other end of the main arm 221, which is opposite to the one end to which the top plate support arm 23 is attached, with the C-arm 25 positioned at a predetermined position.

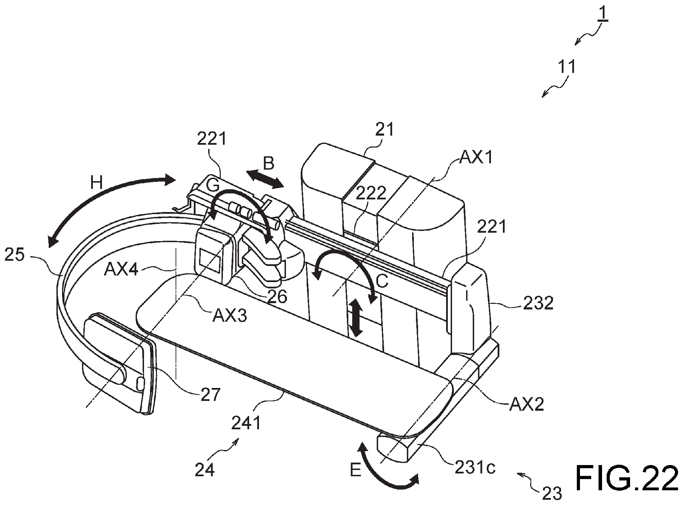

FIG. 22 is a diagram illustrating an example of a case in which the C-arm 25 is moved to a predetermined position in the fourth embodiment. As illustrated in FIG. 22, the control function 411a controls the C-arm support 222 to move the C-arm 25 to the other end. Simultaneously, the control function 411a moves the C-arm support 222 to the other end of the main arm 221, which is opposite to the one end to which the top plate support arm 23 is attached, such that a third axis AX3 connecting the X-ray tube 26 to the X-ray detector 27 is perpendicular to the longitudinal direction of the top plate main body 241 and a movement center axis AX4 of the C-arm 25 in an arc direction (H direction illustrated in FIG. 22) is perpendicular to the top plate 24.

Next, as illustrated in FIG. 21, the control function 411a rotates the main arm 221 and the top plate support arm 23 (step S51). Specifically, the control function 411a controls the power circuitry 29 to rotate the main arm 221 and the C-arm support 222 and the top plate support 231 in the top plate support arm 23. More specifically, the control function 411a rotates the main arm 221, the C-arm support 222, and the top plate support 231 by 180°. As described above, in the support direction change mode, the control function 411a moves the top plate support arm 23 in the left-to-right direction with respect to the base 21 by rotating the main arm 221 and also rotating the top plate support arm 23 about the second axis AX2, which is an axis in the lateral direction of the top plate 24.

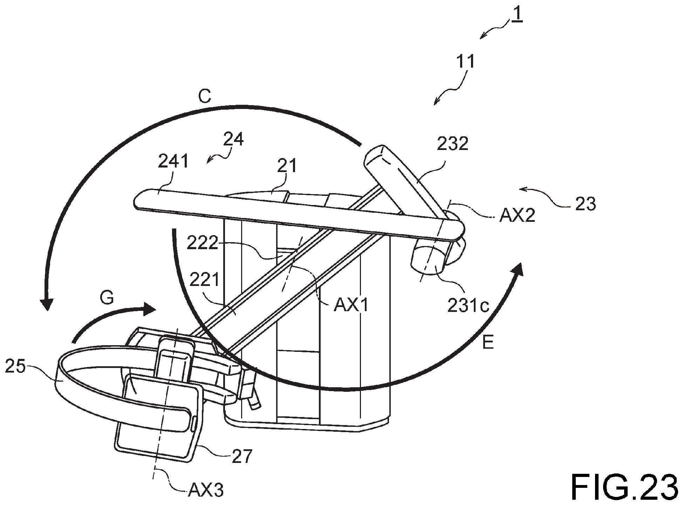

FIG. 23 is a diagram illustrating an example of rotational operation of the main arm 221 and the top plate support arm 23 in the X-ray diagnosis apparatus 1 according to the fourth embodiment. As illustrated in FIG. 23, in the support direction change mode, the control function 411a moves the top plate support arm 23 in the left-to-right direction with respect to the base 21 by rotating the main arm 221 and the top plate support arm 23 in a first rotational direction while rotating the C-arm 25 in a second rotational direction different from the first rotational direction. More specifically, in the example illustrated in FIG. 23, the control function 411a controls the power circuitry 29 to rotate the main arm 221 by 180° in the counterclockwise direction, which is the first rotational direction, around the first axis AX1, rotates the C-arm support 222 by 180° in the clockwise direction, which is the second rotational direction, around the third axis AX3 to rotate the C-arm 25 by 180° in the clockwise direction, and rotates the top plate support 231 of the top plate support arm 23 by 180° in the counterclockwise direction, which is the first rotational direction, around the second axis AX2 to move the top plate support arm 23 in the left-to-right direction with respect to the base 21.

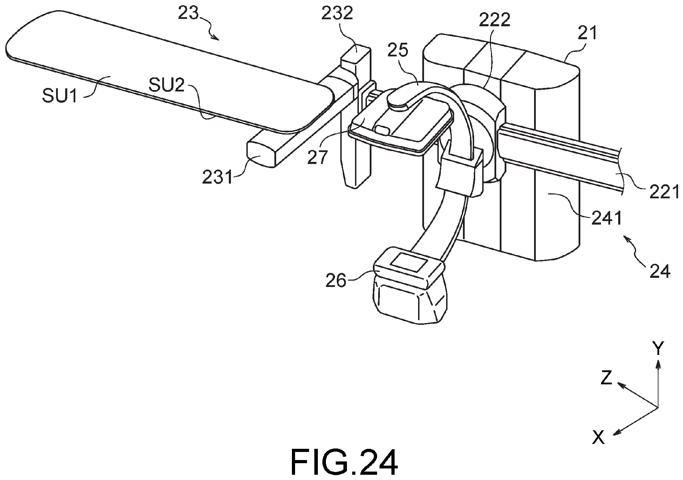

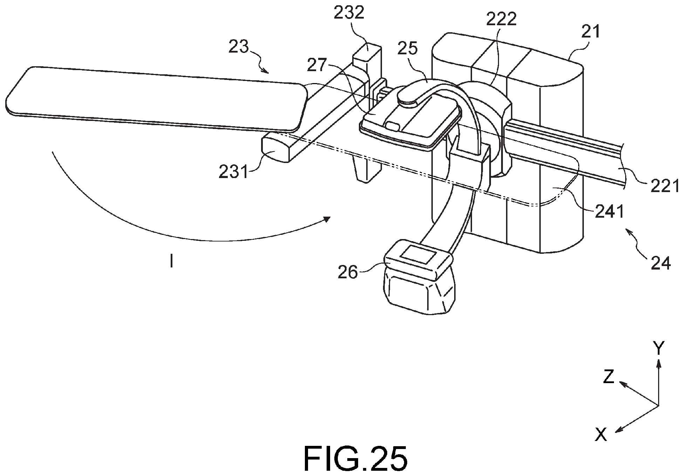

In step S51, the main arm 221 and the top plate support arm 23 are rotated to end the support direction change processing. Thereafter, the operator DO, who is a user, detaches the top plate 24 from the top plate support 231, changes the orientation of the top plate 24, and then changes the support direction of the top plate 24 by attaching the top plate 24 to the top plate support 231 again. FIG. 24 is a diagram illustrating an example of the orientation of the top plate 24 after the support direction change mode is executed in the fourth embodiment. FIG. 25 is a diagram illustrating an example in the middle of changing the orientation of the top plate 24 in the fourth embodiment. As illustrated in FIG. 24, in the fourth embodiment, by executing the support direction change mode, the top plate 24 is supported so as to protrude to the left side of the connecting portion 232, that is, so as to protrude in the +Z direction when the connecting portion 232 is viewed from the front from the side where the top plate support 231 protrudes. Therefore, as illustrated in FIG. 25, the operator DO detaches the top plate 24 from the top plate support 231, rotates (turns) the top plate 24 to the position of the top plate 24 indicated by the two-dot chain line in FIG. 25 so as to rotate in the I direction illustrated in FIG. 25, that is, about the rotation axis parallel to the Y-axis, and replaces the head and foot directions, thereby changing the orientation of the top plate 24 and attaching the top plate 24 to the top plate support 231. Accordingly, the support direction of the top plate 24 is on the left side of the base 21 when the base 21 is viewed from the front.

As described above, in the X-ray diagnosis apparatus 1 according to the fourth embodiment, the support direction change mode can be executed in the state in which the top plate 24 is supported by the top plate support arm 23. Accordingly, the support direction of the top plate 24 can be changed without confirming whether the top plate 24 has been detached, thereby improving user convenience.

In addition, since the control function 411a according to the fourth embodiment executes the support direction change mode in the state where the top plate 24, the attachment orientation of which has been changed, is supported by the top plate support arm, it is possible to reduce the work of retracting the top plate 24 to a position that does not interfere with each part of the X-ray diagnosis apparatus 1 in the support direction change mode after the top plate 24 is detached, thereby making it possible to reduce the number of work steps of the user.

In addition, since the control function 411a according to the fourth embodiment executes the support direction change mode in the state in which the top plate 24, the attachment orientation of which has been changed, is supported by the top plate support arm, it is possible to reduce the number of times the user carries the top plate 24 from the storage position and attaches the top plate 24 after the top plate 24 is detached, the top plate 24 is once stored in the storage position, and the support direction change mode is completed. Therefore, it is possible to improve user convenience.

In the X-ray diagnosis apparatus 1 according to the fourth embodiment, the support direction of the top plate 24 is changed by executing the support direction change mode in the state in which the first top plate surface SU1 of the top plate 24 faces upward. Therefore, when the orientation of the top plate 24 is changed, the orientation of the top plate 24 can be changed without performing an operation such as turning over the first top plate surface SU1 and the second top plate surface SU2 by changing the support direction of the top plate 24 in the state in which the first top plate surface SU1 of the top plate 24 faces downward. Therefore, the support direction of the top plate 24 can be changed without using a guide apparatus for attachment and detachment of the top plate 24 and without increasing the number of work steps of the user.

Note that, in the above-described fourth embodiment, in the support direction change mode, the control function 411a moves the top plate support arm 23 in the left-to-right direction with respect to the base 21 by rotating the main arm 221 and the top plate support arm 23 in the first rotational direction while rotating the C-arm 25 in the second rotational direction different from the first rotational direction, but the present invention is not limited thereto. For example, in the support direction change mode, the control function 411a may move the top plate support arm 23 in the left-to-right direction with respect to the base 21 by rotating the main arm 221 in the first rotational direction while rotating the C-arm 25 and the top plate support arm 23 in the second rotational direction different from the first rotational direction. More specifically, the control function 411a controls the power circuitry 29 to rotate the main arm 221 by 180° in the counterclockwise direction, which is the first rotational direction, around the first axis AX1, to rotate the C-arm support 222 by 180° in the clockwise direction, which is the second rotational direction, around the third axis AX3 to rotate the C-arm 25 by 180° in the clockwise direction, and to rotate the top plate support 231 of the top plate support arm 23 by 180° in the clockwise direction, which is the second rotational direction, around the second axis AX2, thereby moving the top plate support arm 23 in the left-to-right direction with respect to the base 21.

In the X-ray diagnosis apparatus 1 according to the fourth embodiment, it has been described that, after the support direction change mode is executed, the top plate 24 is rotated (turned) to the position of the top plate 24 indicated by the two-dot chain line in FIG. 25 so that the operator DO rotates in the I direction illustrated in FIG. 25, that is, about the rotation axis parallel to the Y-axis, and the orientation of the top plate 24 is changed by switching the head and foot directions of the top plate 24. However, a mechanism for changing the orientation of the top plate 24 may be provided, and the control function 411a may change the orientation of the top plate 24 by controlling the mechanism for changing the orientation of the top plate 24.

### [Fifth Embodiment]

In the X-ray diagnosis apparatus 1 according to the above-described first embodiment, it is also possible to execute an imaging mode for imaging the subject P seated in the wheelchair without detachment of the top plate 24. Note that, although the configuration of the X-ray diagnosis apparatus according to the fifth embodiment is not illustrated, the control function in the processing circuitry 41 according to the present embodiment is different from that of the above-described first embodiment, and thus the control function will be referred to as a control function 411b. Note that configurations and functions other than the control function 411b are the same as those of the first embodiment, and thus the description thereof will be omitted.

The control function 411b according to the fifth embodiment executes a top plate retraction mode, which is an imaging mode for retracting the top plate 24 upward. In the present embodiment, the control function 411b executes the top plate retraction mode in a state in which the top plate 24 is supported by the top plate support arm 23. Specifically, in the top plate retraction mode, the control function 411b executes rotation of the main arm 221 in the lateral direction of the top plate 24 and rotation of the top plate support arm 23 to move the top plate 24 upward. The top plate retraction mode corresponds to a second control mode in the present embodiment.

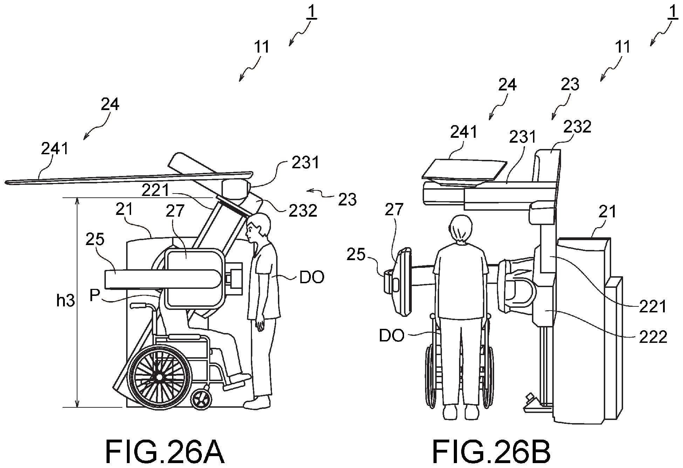

FIG. 26 is a diagram illustrating an example of the position of the top plate 24 and the position of the C-arm 25 after execution of the top plate retraction mode in the fifth embodiment. FIG. 26A is a diagram illustrating an example of the position of the top plate 24 and the position of the C-arm 25 when the base 21 after execution of the top plate retraction mode is viewed from the front in the fifth embodiment, and FIG. 26B is a diagram illustrating an example of the position of the top plate 24 and the position of the C-arm 25 when the base 21 after execution of the top plate retraction mode is viewed from the side in the fifth embodiment.

As illustrated in FIG. 26, the control function 411b executes the top plate retraction mode, and the main arm 221 and the top plate support 231 of the top plate support arm 23 rotate, so that the top plate 24 moves above the operator DO. As illustrated in FIG. 26, a distance h3 between the lowermost end of the top plate support arm 23 and the floor surface when the top plate 24 moves upward is, for example, 2000 mm or more. In the example illustrated in FIG. 26, the control function 411b moves the top plate 24 above the floor surface such that the top plate 24 is held at a position higher than the base 21 when the top plate retraction mode is executed. In addition, in order to image the subject P seated in the wheelchair, the control function 411b rotates the C-arm support 222 and moves the C-arm support 222 along the main arm 221.

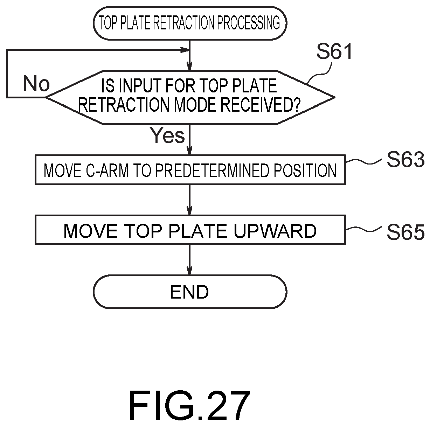

FIG. 27 is a flowchart for description of the top plate retraction processing executed in the X-ray diagnosis apparatus 1 according to the fifth embodiment. In the top plate retraction processing, the top plate retraction mode is received, the C-arm 25 is moved to a predetermined position, and the top plate 24 is moved upward. For example, the top plate retraction processing is processing executed when the input for the top plate retraction mode is received.

As illustrated in FIG. 27, first, the control function 411b in the processing circuitry 41 of the X-ray diagnosis apparatus 1 determines whether an input for the top plate retraction mode has been received (step S61). Specifically, the control function 411b determines whether the input for the top plate retraction mode has been received from the operator via the input interface 45. When the control function 411b has not received the input for the top plate retraction mode in step S61 (step S61: No), the control function 411b waits until the input for the top plate retraction mode is received.

Next, as illustrated in FIG. 27, the control function 411b moves the C-arm 25 to a predetermined position (step S63). Specifically, the control function 411b controls the C-arm support 222 to move the C-arm support 222 to the other end of the main arm 221, which is opposite to the one end to which the top plate support arm 23 is attached, with the C-arm 25 positioned at a predetermined position.

Next, as illustrated in FIG. 27, the control function 411b moves the top plate 24 upward (step S65). Specifically, the control function 411b controls the main arm 221 and the top plate support 231 in the top plate support arm 23 to move the top plate 24 upward. More specifically, the control function 411b moves the top plate 24 upward by rotating the main arm 221 counterclockwise about the first axis AX1 and rotating the top plate support arm 23 clockwise about the second axis AX2. As described above, in the top plate retraction mode, the control function 411b moves the top plate 24 upward by rotating the main arm 221 in the first rotational direction while rotating the C-arm 25 and the top plate support arm 23 in the second rotational direction different from the first rotational direction.

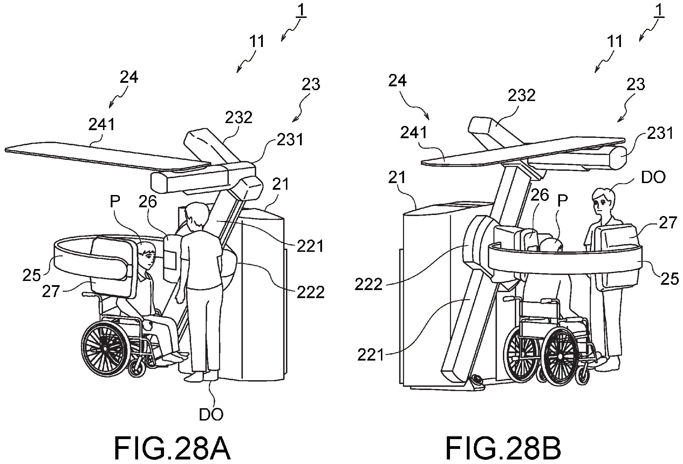

In step S65, the top plate retraction processing is ended by moving the top plate 24 upward. Thereafter, the operator DO, who is a user, introduces the subject P seated in the wheelchair into the imaging apparatus 11, and performs imaging by performing alignment of the C-arm 25. FIG. 28 is a diagram illustrating an example of a positional relationship between the top plate 24 and the C-arm 25 after introduction of the subject P in the fifth embodiment. As illustrated in FIG. 28, as the top plate 24 moves upward, there is a space for the operator DO, who is a user, to align the subject P without attachment and detachment of the top plate, and the subject P is positioned in the imaging apparatus 11 in a state of being seated in a wheelchair.

As described above, in the X-ray diagnosis apparatus 1 according to the fifth embodiment, the top plate 24 is moved upward by rotating the main arm 221 and rotating the top plate support 231 of the top plate support arm 23, thereby enabling the subject P to be positioned in the imaging apparatus 11 without attachment and detachment of the top plate 24. Consequently, the support state of the top plate can be made changeable, and user convenience can be improved while an efficient inspection workflow is achieved.

As a comparative example with respect to the X-ray diagnosis apparatus 1 according to the fifth embodiment, there is an X-ray diagnosis apparatus 1 capable of imaging a patient seated in a wheelchair without detachment of the top plate when imaging the patient seated in the wheelchair. However, there is a problem in that X-rays emitted from the X-ray tube 26 enter the X-ray detector through the top plate 24, and are affected by the aluminum equivalent of the top plate 24.

On the other hand, according to the X-ray diagnosis apparatus 1 of the fifth embodiment, by rotating the main arm 221 and rotating the top plate support 231 of the top plate support arm 23, the top plate 24 is moved upward, and the subject P can be positioned in the imaging apparatus 11 without attachment and detachment of the top plate 24. Accordingly, imaging can be performed without being affected by the aluminum equivalent of the top plate 24.

Note that, in step S63 in the above-described top plate retraction processing, the predetermined position is not limited thereto. That is, the predetermined position is the other end that is opposite to the one end to which the top plate support arm 23 is attached, but the present invention is not limited thereto. That is, in step S65, the predetermined position is arbitrary as long as the top plate 24 can be moved upward. For example, the C-arm 25 may be disposed to face the second top plate surface SU2 of the top plate main body 241, and the C-arm 25 may be moved such that a line connecting the X-ray tube 26 to the X-ray detector 27 is parallel to the first top plate surface of the top plate main body 241.

Further, in step S63 in the above-described top plate retraction processing, the control function 411b executes the rotation of the top plate 24 of the main arm 221 in the lateral direction and the rotation of the top plate support arm 23 to move the top plate 24 upward in the top plate retraction mode, but the control function 411b may execute the rotation of the top plate 24 of the main arm 221 in the lateral direction and the rotation of the top plate support arm 23 in the top plate retraction mode, and may also execute the movement of the C-arm 25 along the main arm 221 to move the top plate 24 upward.

### [Sixth Embodiment]

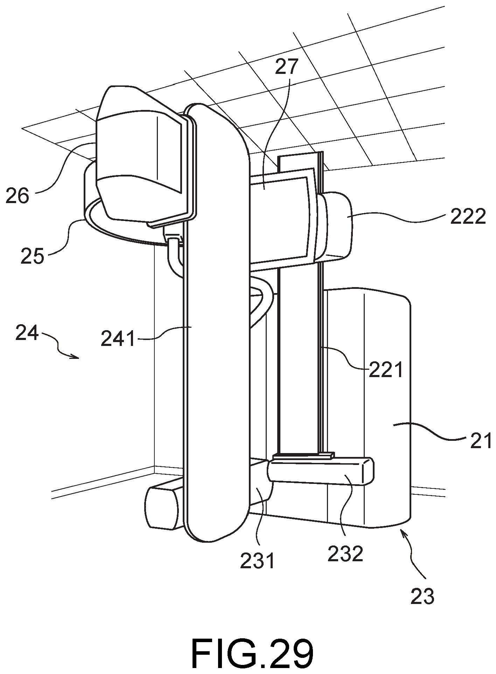

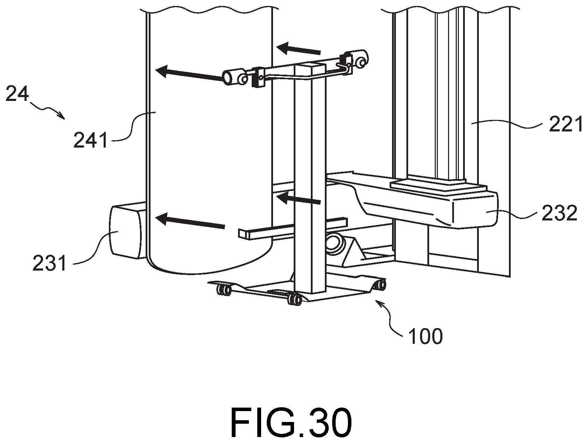

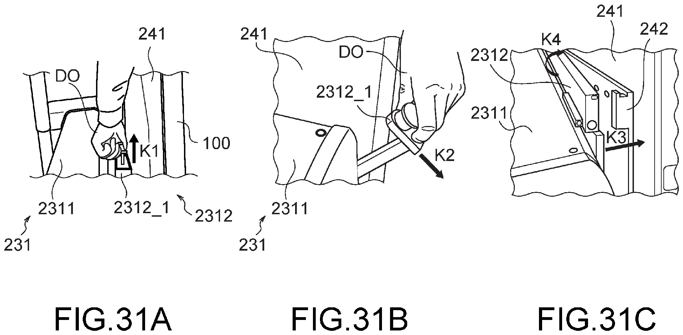

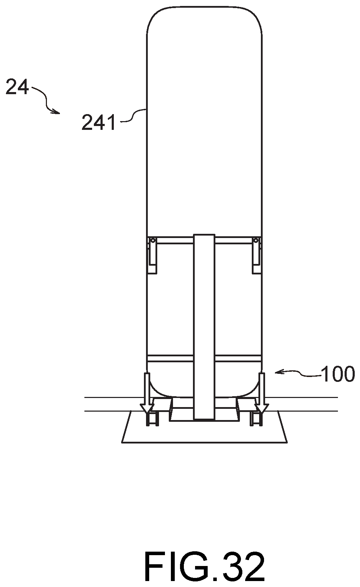

In the first to fifth embodiments described above, a cart may be used to attachment and detachment of the top plate 24. An example of a method of attachment and detachment of the top plate 24 will be described with reference to FIGS. 29 to 32. FIG. 29 is a diagram illustrating an example of an aspect in which the top plate 24 is disposed in a detachable state. FIG. 30 is a diagram illustrating an example of a state in which a cart for enabling the top plate 24 to move is attached to the top plate 24 in the sixth embodiment. FIG. 31 is a diagram illustrating an example of a method of detaching the top plate 24 from the top plate support 231 in the sixth embodiment, FIG. 31A is a diagram illustrating a state in which a first operation by a user has been performed on the fixing mechanism 2312 in the sixth embodiment, FIG. 31B is a diagram illustrating a state in which a second operation by the user has been performed on the fixing mechanism 2312, and FIG. 31C is a diagram illustrating a state in which a third operation by the user has been performed on the fixing mechanism 2312. FIG. 32 is a diagram illustrating an example of a state in which the top plate 24 is detached from the top plate support 231 in the sixth embodiment.

As illustrated in FIG. 29, the control function 411 controls the power circuitry 29 such that the top plate 24 is substantially perpendicular to the floor surface based on an instruction from the operator DO, who is a user, via the input interface 45. The control function 411 controls the power circuitry 29 such that the C-arm 25 is disposed to face the second top plate surface SU2 of the top plate main body 241, and the X-ray tube 26 and the X-ray detector 27 are disposed such that a line connecting the X-ray tube 26 to the X-ray detector 27 is parallel to the first top plate surface of the top plate main body 241.

Next, as illustrated in FIG. 30, the user attaches a cart 100 for enabling the top plate 24 to move from the first top plate surface SU1 side of the top plate 24, on which the subject P is placed, to the top plate 24.

Next, as illustrated in FIG. 31A, as a first operation, the operator DO, who is a user, performs an operation of lifting a locking mechanism 2311_1 included in the fixing mechanism 2312 upward (a K1 direction illustrated in FIG. 31A) to release the lock of the fixing mechanism 2312 from the attachment portion 242 of the top plate 24. Next, as illustrated in FIG. 31B, in a state in which the operator DO, who is the user, releases the lock of the fixing mechanism 2312 with respect to the attachment portion 242 of the top plate 24 illustrated in FIG. 31A, as a second operation, the operator DO moves the locking mechanism 2311_1 downward (a K2 direction illustrated in FIG. 31B) to detach the locking mechanism 2311_1 from the attachment portion 242. Next, as illustrated in FIG. 31C, as a third operation, the operator DO, who is the user, moves the top plate main body 241 in a K3 direction and a K4 direction while gripping the top plate main body 241 or the cart 100 to which the top plate main body 241 is attached, thereby detaching the attachment portion 242 of the top plate 24 from the fixing mechanism 2312 of the top plate support 231. Then, as illustrated in FIG. 32, the operator DO, who is the user, moves the cart 100 to which the top plate 24 is attached.

Note that, although the method of detaching the top plate 24 from the top plate support arm 23 has been described with reference to FIGS. 29 to 32, the method of attaching the top plate 24 to the top plate support arm 23 is substantially the opposite procedure. That is, the operator DO, who is the user, moves the cart 100 to which the top plate 24 is attached to the vicinity of the top plate support arm 23. Next, the operator DO, who is the user, attaches the attachment portion 242 of the top plate 24 to the fixing mechanism 2312 of the top plate support 231 so as to bring the top plate main body 241 close to the top plate support arm 23 while gripping the top plate main body 241 or the cart 100 to which the top plate main body 241 is attached. Next, the operator DO, who is a user, moves the locking mechanism 2311_1 upward to attach the locking mechanism 2311_1 to the attachment portion 242, and locks and fixes the attachment portion 242 of the top plate 24 to the fixing mechanism 2312. As described above, the operator DO, who is the user, attaches the top plate 24 to the top plate support arm 23.

In the above-described first embodiment, attachment and detachment of the top plate 24 is performed by using the cart 100, but the present invention is not limited thereto. That is, a method of attaching and detaching the top plate 24 is arbitrary, and for example, the top plate 24 may be detached from the top plate support arm 23 or may be attached to the top plate support arm 23 by a plurality of users.

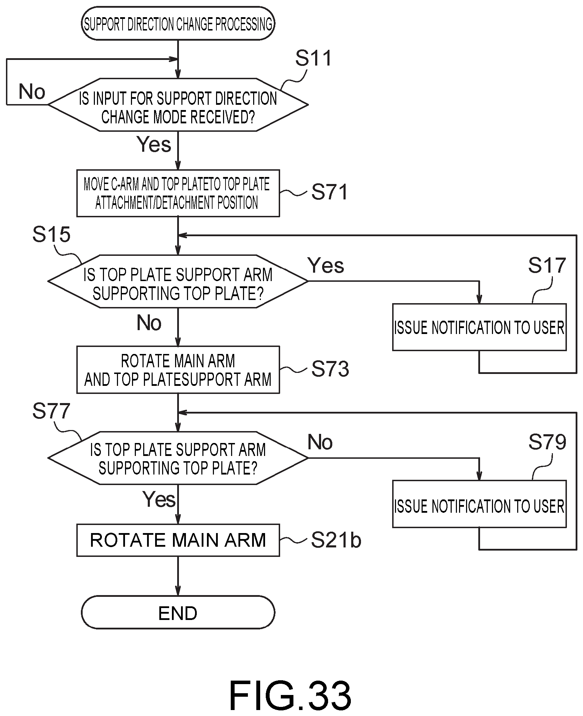

FIG. 33 is a flowchart for description of support direction change processing executed in the X-ray diagnosis apparatus 1 according to the sixth embodiment, and is a diagram corresponding to FIG. 5. In this support direction change processing, an input for a support direction change mode is received, the C-arm 25 and the top plate 24 are moved to the top plate attachment/detachment position, a detection result of the top plate 24 is acquired, the C-arm 25 is moved, the main arm 221 is rotated, and the top plate support arm 23 is rotated. For example, the support direction change processing is processing executed when the input for the support direction change mode is received. Note that processing of step S11 is the same as that in FIG. 5, and thus the description thereof will be omitted.

Next, as illustrated in FIG. 33, the control function 411 in the processing circuitry 41 of the X-ray diagnosis apparatus 1 moves the C-arm 25 and the top plate 24 to the top plate attachment/detachment position (step S71). Specifically, the control function 411 controls the main arm 221 and the C-arm support 222 to move the C-arm 25 and the top plate 24 to the top plate attachment/detachment position, which is a position for attachment/detachment of the top plate 24. More specifically, as illustrated in FIG. 29, the control function 411 moves the C-arm 25 and the top plate 24 such that the top plate 24 is substantially perpendicular to the floor surface, the C-arm 25 is disposed to face the second top plate surface SU2 of the top plate main body 241, and a line connecting the X-ray tube 26 to the X-ray detector 27 is parallel to the first top plate surface of the top plate main body 241. Note that the processing from step S13 to step S17 after step S71 is the same as that in FIG. 5, and thus the description thereof will be omitted.

Next, as illustrated in FIG. 33, the control function 411 in the processing circuitry 41 of the X-ray diagnosis apparatus 1 rotates the main arm 221 and the top plate support arm 23 (step S73). Specifically, the control function 411 controls the power circuitry 29 to rotate the C-arm support 222 and the top plate support arm 23 by 180°. More specifically, the control function 411 rotates the C-arm support 222 by 180° about the third axis to rotate the C-arm 25 by 180°, and also rotates the top plate support 231 of the top plate support arm 23 by 180° about the second axis AX2.

Next, as illustrated in FIG. 33, the control function 411 acquires a detection result of the top plate 24 (step S75). Specifically, the control function 411 acquires the detection result of the top plate 24 output from the top plate detection sensor 2313.

Next, as illustrated in FIG. 33, the control function 411 determines whether the top plate 24 is attached (step S77). Specifically, the control function 411 determines whether the top plate 24 is attached based on the detection result of the top plate 24 acquired in step S75.

Then, in step S77, when the top plate 24 is not attached (step S77: No), the control function 411 issues a notification to a user (step S79). Specifically, the control function 411 notifies the user of information regarding attachment of the top plate 24 via the display 46. Then, the processing returns to step S75, and the processing of steps S75 to S79 is repeated until the operator DO, which is the user, detects that the top plate 24 is attached to the top plate support 231.

On the other hand, in step S77, when the top plate 24 is attached (step S77: Yes), the control function 411 rotates the main arm 221 (step S21b). Specifically, the control function 411 controls the main arm 221 via the power circuitry 29 to rotate the main arm 221 such that the support direction of the top plate 24 is to the left when the base 21 is viewed from the front.

In step S21a, the main arm 221 is rotated to end the support direction change processing.

As described above, in the X-ray diagnosis apparatus 1 according to the sixth embodiment, even when the top plate 24 is detached using the cart 100, the user can detach the top plate 24 by moving the C-arm 25 and the top plate 24 to the top plate attachment/detachment position in the support direction change processing, and the C-arm support and the top plate support arm are rotated after the user detaches the top plate 24 so that the top plate 24 can be attached. Therefore, the support state of the top plate can be changed according to the procedure.

### [Seventh Embodiment]

In the X-ray diagnosis apparatus 1 according to the above-described first to sixth embodiments, the control of at least one of the main arm 221 and the top plate support arm 23 may be determined based on dimensional information regarding dimensions of a space in which the X-ray diagnosis apparatus is disposed. Hereinafter, a case in which this modification is applied to the fourth embodiment will be referred to as a seventh embodiment, and portions different from those of the fourth embodiment will be described. Note that this modification can also be applied to the first to third, fifth, and sixth embodiments.

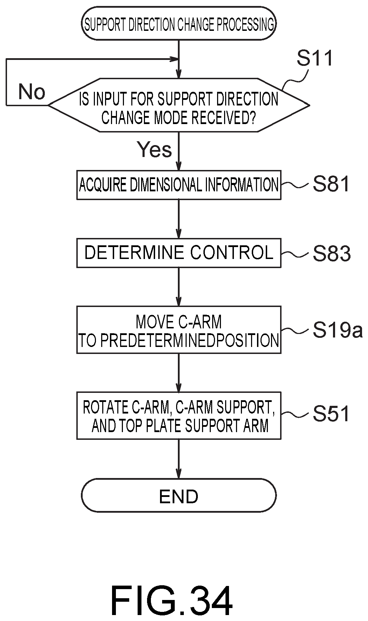

FIG. 34 is a flowchart for description of support direction change processing executed in the X-ray diagnosis apparatus 1 according to the seventh embodiment, and is a diagram corresponding to FIG. 21. In the support direction change processing according to the seventh embodiment, an input for a support direction change mode is received, dimensional information is acquired, control is determined, the C-arm 25 is moved, and the C-arm 25, the main arm 221, and the top plate support arm 23 are rotated. For example, the support direction change processing is processing executed when the input for the support direction change mode is received. Note that the processing in step S11 illustrated in FIG. 34 is the same as that in FIG. 5, and thus the description thereof will be omitted.

Upon receiving an input for a support direction change mode in step S11 (step S11: Yes), the control function 411a in the processing circuitry 41 of the image processing apparatus 12 acquires dimensional information (step S81). Specifically, the control function 411a acquires the dimensional information from the storage 42. Here, the dimensional information is information regarding dimensions of a space in which the X-ray diagnosis apparatus 1 is disposed. The dimensional information includes, for example, a width, a depth, and a height of the space in which the X-ray diagnosis apparatus 1 is disposed. In addition, the dimensional information includes the arrangement position of the X-ray diagnosis apparatus 1 in the space in which the X-ray diagnosis apparatus is arranged and the apparatus size of the X-ray diagnosis apparatus. Note that the dimensional information may be, for example, a 3D model of the space in which the X-ray diagnosis apparatus is disposed.

Note that, in step S81, the control function 411a acquires the dimensional information from the storage 42, but a method of acquiring the dimensional information is not limited thereto. That is, the method of acquiring the dimensional information is arbitrary, and the control function 411a may acquire the dimensional information using, for example, an imaging apparatus such as a camera or a distance sensor.

Next, as illustrated in FIG. 34, the control function 411a determines control (step S83). Specifically, the control function 411a determines control of at least one of the main arm 221 and the top plate support arm 23 according to the dimensional information acquired in step S81. More specifically, the control function 411a according to the present embodiment determines control amounts of the main arm 221 and the top plate support arm 23 such that the main arm 221, the top plate support arm 23, the C-arm 25, and the top plate 24 do not interfere with the floor, the wall, and the ceiling according to the dimensional information, thereby determining at least one control of the main arm 221 and the top plate support arm 23. Note that the processing in steps S19a and S51 after step S83 is the same as that in FIG. 21, and thus the description thereof will be omitted.

As described above, in the X-ray diagnosis apparatus 1 according to the seventh embodiment, the control function 411a determines the control of at least one of the main arm 221 and the top plate support arm 23 according to the dimensional information. Therefore, the control amount of at least one of the main arm 221 and the top plate support arm 23 according to the space in which the X-ray diagnosis apparatus 1 is disposed can be determined, and thus at least one of the main arm 221 and the top plate support arm 23 can be controlled so as not to interfere with the floor, the wall, the ceiling, and the like.

Note that, in the X-ray diagnosis apparatus 1 according to the above-described seventh embodiment, instead of determining the control of the main arm 221 and the top plate support arm 23 such that the main arm 221, the top plate support arm 23, the C-arm 25, and the top plate 24 do not interfere with the floor, the wall, and the ceiling according to the dimensional information, the control of at least one of the main arm 221 and the top plate support arm 23 may be determined by selecting a preset related to the control of at least one of the main arm 221 and the top plate support arm 23 based on the dimensional information. Specifically, the control function 411a may determine the control of at least one of the main arm 221 and the top plate support arm 23 by selecting a preset in which each of the main arm 221, the top plate support arm 23, the C-arm 25, and the top plate 24 does not interfere with the floor, the wall, and the ceiling from a plurality of presets stored in the storage 42 based on the dimensional information.

In addition, the control function 411a may store the determined control amount or the selected preset in the storage 42 in step S83, and when the input for the support direction change mode is received in step S11 of the support direction change processing illustrated in FIG. 34 and the determined control amount or the selected preset is stored in the storage 42, the control function 411a may acquire the determined control amount or the selected preset from the storage 42 and may control at least one of the main arm 221 and the top plate support arm 23 based on the determined control amount or the selected preset. In this case, the processing in steps S81 and S83 of the support direction change processing illustrated in FIG. 34 is omitted.

### [Other Modifications]

In step S11 of the support direction change processing executed in the X-ray diagnosis apparatus 1 according to the above-described first to seventh embodiments, the control function 411 receives the input for the support direction change mode from the operator via the input interface 45, but a method in which the control function 411 receives the support direction change mode is not limited to a case in which the operator performs the input via the input interface 45. That is, the method in which the control function 411 receives the support direction change mode is arbitrary, and for example, the control function 411 may acquire imaging conditions including information regarding the support direction of the top plate 24 via a communication interface, and may change the support direction of the top plate 24 based on the information regarding the support direction of the top plate 24.

In the X-ray diagnosis apparatus 1 according to the above-described first to seventh embodiments, the top plate supports 231, 231a, and 231b are described as including a fixing mechanism, but are not limited to the fixing mechanism. The top plate supports 231, 231a, and 231b may include, for example, a connection mechanism or an attachment/detachment mechanism.

Furthermore, in the above-described first modification, the control function 411 may acquire information regarding the support direction of the top plate 24 after the support direction change mode, may determine whether the attachment direction of the top plate 24 is correct based on the information regarding the support direction of the top plate 24, and may issue a notification when the attachment direction of the top plate 24 is incorrect. Specifically, the X-ray diagnosis apparatus 1 may include a top plate attachment direction detection sensor formed by a camera, an optical sensor, a weight sensor, a micro switch, and the like and configured to detect the attachment direction of the top plate 24. After executing the support direction change mode, the control function 411 may determine whether the attachment direction of the top plate 24 is correct based on a detection result of a micro switch provided on the top plate support arm 23 as the top plate attachment direction detection sensor. When the attachment direction of the top plate 24 is correct, the control function 411 may operate the main arm 221 and the like by executing various imaging modes. Conversely, when the attachment direction of the top plate 24 is incorrect, the control function 411 may issue a notification indicating that the attachment direction of the top plate 24 is incorrect. As a result, it is possible to prevent the main arm 221 and the like from operating due to the control function 411 executing various imaging modes in a state in which the attachment direction of the top plate 24 is incorrect.

Note that the word "processor" used in above descriptions means circuits such as, for example, a Central Processing Unit (CPU), a Graphics Processing Unit (GPU), an Application Specific Integrated Circuit (ASIC), a programmable logic device (for example, a Simple Programmable Logic Apparatus (SPLD), a Complex Programmable Logic Apparatus (CPLD), and a Field Programmable Gate Array (FPGA)). The processor executes functions by reading and executing programs stored in the storage 42. Note that programs may be configured to be directly integrated in the processor instead of being storing in the storage 42. In this case, the processor realizes functions by reading and executing programs stored in the circuitry. Note that the processor is not limited to the case arranged as a single processor circuit, but may be configured as a single processor by combining a plurality of independent circuits to realize functions. Furthermore, a plurality of component elements in Fig.1 may be integrated into one processor to realize the functions.

According to at least one embodiment described above, the support state of the top plate can be changed.

While certain embodiments have been described, these embodiments have been presented by way of example only and are not intended to limit the scope of the inventions. The embodiments may be in a variety of other forms. Furthermore, various omissions, substitutions and changes may be made without departing from the scope of the invention as defined by the appended claims. The embodiments and their modifications are included in the scope and the subject matter of the invention, and at the same time included in the scope of the claimed inventions.

## Claims

1. An X-ray diagnosis apparatus comprising:
a C-arm configured to support an X-ray tube and an X-ray detector;
a C-arm support configured to rotatably support the C-arm;
a main arm configured to be supported by a base, the main arm being configured to support the C-arm support;
(a) a top plate support arm configured to be rotatable about an axis in a lateral direction of a top plate, the top plate support arm being supported by the main arm and being configured to support the top plate on which a subject is placed or (b) a top plate support arm configured to be supported by the main arm and to support a top plate on which a subject is placed, the top plate support arm including a plurality of support surfaces for supporting the top plate; and
processing circuitry configured to rotate the main arm by controlling the main arm.

2. The X-ray diagnosis apparatus of Claim 1,
wherein (a) the top plate support arm includes:
a top plate support configured to be rotatable in the lateral direction of the top plate, the top plate support being configured to support the top plate; and
a connecting portion configured to connect the top plate support to the main arm.

3. The X-ray diagnosis apparatus of Claim 1,
wherein the processing circuitry is further configured to, in a first control mode for changing a support direction of the top plate, change the support direction of the top plate by rotating the main arm to move the top plate support arm in a left-to-right direction with respect to the base.

4. The X-ray diagnosis apparatus of Claim 3,
wherein the processing circuitry is further configured to, in the first control mode, move the top plate support arm in the left-to-right direction with respect to the base by rotating the main arm by 180°.

5. The X-ray diagnosis apparatus of Claim 3,
wherein the processing circuitry is further configured to, in the first control mode, move the top plate support arm in the left-to-right direction with respect to the base by rotating the main arm and rotating the top plate support arm about the axis in the lateral direction of the top plate.

6. The X-ray diagnosis apparatus of Claim 3,
wherein the processing circuitry is further configured to execute the first control mode in a state in which the top plate is supported by the top plate support arm.

7. The X-ray diagnosis apparatus of Claim 1,
wherein the processing circuitry is further configured to:
determine whether the top plate support arm supports the top plate; and
rotate the main arm when the top plate support arm does not support the top plate.

8. The X-ray diagnosis apparatus of Claim 3,
wherein the processing circuitry is further configured to, in the first control mode, move at least one of the main arm and the top plate support arm in a vertical direction to cause a height of the top plate after execution of the first control mode to substantially match a height of the top plate before the execution thereof.

9. The X-ray diagnosis apparatus of Claim 1,
wherein the processing circuitry is further configured to, in a second control mode for retracting the top plate upward, move the top plate upward by executing rotation of the main arm in the lateral direction of the top plate and the rotation of the top plate support arm in a state in which the top plate is supported by the top plate support arm.

10. The X-ray diagnosis apparatus of Claim 9,
wherein the processing circuitry is further configured to, upon execution of the second control mode, move the top plate upward such that the top plate is held at a position higher than the base.

11. The X-ray diagnosis apparatus of Claim 1,
wherein the plurality of support surfaces are provided symmetrically in a direction orthogonal to a longitudinal direction and the lateral direction of the top plate.

12. The X-ray diagnosis apparatus of Claim 1,
wherein (b)the top plate support arm includes a fixer configured to detachably fix the top plate,
wherein the top plate support arm includes, as the plurality of support surfaces, a first support surface of the top plate support arm and a second support surface of the top plate support arm, and
wherein the top plate support arm has an insertion hole formed therein and configured for the fixer to be inserted thereinto, the fixer being configured to fix the top plate supported by the first support surface when the top plate is supported by the first support surface, to fix the top plate supported by the second support surface when the top plate is supported by the second support surface, and to detachably fix the top plate.

13. A method of controlling an X-ray diagnosis apparatus,
the X-ray diagnosis apparatus including:
a C-arm configured to support an X-ray tube and an X-ray detector;
a C-arm support configured to rotatably support the C-arm;
a main arm configured to be supported by a base, the main arm being configured to support the C-arm support;
(a) a top plate support arm configured to be rotatable about an axis in a lateral direction of a top plate, the top plate support arm being supported by the main arm and being configured to support the top plate on which a subject is placed or (b) a top plate support arm configured to be supported by the main arm and to support a top plate on which a subject is placed, the top plate support arm including a plurality of support surfaces for supporting the top plate,
the method comprising rotating the main arm by controlling the main arm.
